# EUROPEAN PATENT APPLICATION

(11) **EP 4 850 597 A1**
(43) Date of publication of application: **09.12.2026**
(21) Application number: 25773198.4
(22) Date of filing: 20.03.2025
(51) Int. Cl.: A61B 5/021

(54) **BLOOD PRESSURE MEASUREMENT METHOD, ELECTRONIC DEVICE, AND STORAGE MEDIUM**

(30) Priority: 20.03.2024 CN 202410327076; 18.03.2025 WO PCT/CN2025/083171
(71) Applicant: HUAWEI TECHNOLOGIES CO., LTD., Shenzhen, Guangdong 518129 (CN)
(72) Inventor: JIN, Junye, Shenzhen, Guangdong 518129 (CN); HUANG, Yadong, Shenzhen, Guangdong 518129 (CN); ZHANG, Ze, Shenzhen, Guangdong 518129 (CN); ZHANG, Lei, Shenzhen, Guangdong 518129 (CN); LU, Shiqiang, Shenzhen, Guangdong 518129 (CN)
(74) Representative: Gill Jennings & Every LLP
(86) International application number: PCT/CN2025/083745
(87) International publication number: WO 2025/195456

(57) **Abstract**

This application provides a blood pressure measurement method, an electronic device, and a storage medium. A wearable device includes a first heart rate sensor and a second heart rate sensor, a watch body of the wearable device includes a case back and a display surface, a first button is disposed on a side surface of the watch body, the first heart rate sensor is disposed on the first button, and the second heart rate sensor is disposed on the case back. The wearable device obtains first information, where the first information includes one or more of the following: user information, environment information, and motion data. The wearable device determines a first blood pressure measurement solution based on the first information, where the first blood pressure measurement solution includes at least one of the following blood pressure measurement methods: measuring blood pressure based on first heart rate data collected by the first heart rate sensor, and measuring blood pressure based on second heart rate data collected by the second heart rate sensor. This improves accuracy of measuring blood pressure by the wearable device and comfort of a user during blood pressure measurement.

## Description

This application claims priorities to Chinese Patent Application No. 202410327076.9, filed with the China National Intellectual Property Administration on March 20, 2024 and entitled "BLOOD PRESSURE MEASUREMENT METHOD, ELECTRONIC DEVICE, AND STORAGE MEDIUM", and to International Patent Application No. PCT/CN2025/083171, filed on March 18, 2025 and entitled "BLOOD PRESSURE MEASUREMENT METHOD, ELECTRONIC DEVICE, AND STORAGE MEDIUM", both of which are incorporated herein by reference in their entireties.

### TECHNICAL FIELD

This application relates to the field of terminal technologies, and in particular, to a blood pressure measurement method, an electronic device, and a storage medium.

### BACKGROUND

With improvement of living standards, people's physical health is increasingly valued, and there are increasingly more hypertension users. Therefore, it is of great significance to monitor blood pressure.

To facilitate blood pressure measurement of the users, wearable devices already have blood pressure measurement components, and the users may measure blood pressure by using the wearable devices worn by the users. When the user needs to measure blood pressure, the user may actively perform an operation on the wearable device, to trigger the wearable device to start to measure blood pressure by using the blood pressure measurement components. However, the user needs to actively perform the operation, and the operation is complex. How to improve convenience of measuring blood pressure by using the wearable device is to be further studied.

### SUMMARY

This application provides a blood pressure measurement method, an electronic device, and a storage medium, so that blood pressure can be automatically measured without a user operation. This further improves accuracy of blood pressure measured by a wearable device and comfort of a user during blood pressure measurement.

According to a first aspect, this application provides a blood pressure measurement method. A wearable device includes a bulb, a barometric pressure sensor, a first heart rate sensor, and a second heart rate sensor, and the bulb is connected to the barometric pressure sensor. The wearable device further includes a watch body, the watch body includes a case back and a display surface, a first button is disposed on a side surface of the watch body, the first heart rate sensor is disposed on the first button, and the second heart rate sensor is disposed on the case back. The method includes: The wearable device obtains first information, where the first information includes one or more of the following: user information, environment information, and motion data. The wearable device determines a first blood pressure measurement solution based on the first information, where the first blood pressure measurement solution includes at least one of the following blood pressure measurement methods: a first blood pressure measurement method for measuring blood pressure based on the bulb and the barometric pressure sensor, a second blood pressure measurement method for measuring blood pressure based on first heart rate data collected by the first heart rate sensor, and a third blood pressure measurement method for measuring blood pressure based on second heart rate data collected by the second heart rate sensor.

Optionally, the wearable device is a watch or a band.

Optionally, the wearable device may further include a pressure sensor and an electrocardiogram sensor. The pressure sensor is disposed on the first button, the electrocardiogram sensor includes a plurality of electrodes, and the plurality of electrodes are disposed on a surface of the first button and/or the case back of the watch body. Measuring the blood pressure based on the first heart rate data collected by the first heart rate sensor specifically includes: measuring the blood pressure based on the first heart rate data collected by the first heart rate sensor, first pressure data collected by the pressure sensor, and electrocardiogram data collected by the electrocardiogram sensor. Therefore, in the second blood pressure measurement method, a blood pressure value of a user may be obtained based on a plurality of pieces of data collected by a plurality of components, to improve accuracy of blood pressure measured based on the second blood pressure measurement method. Optionally, the electrocardiogram sensor may be an ECG sensor.

Optionally, the heart rate sensor may be any one or a combination of a PPG sensor, a laser sensor, an optical-to-electrical conversion sensor, an ultrasonic sensor, or a magnetic induction sensor.

Optionally, when blood pressure is measured based on the first blood pressure measurement method, the wearable device may prompt the user to keep the wrist wearing the wearable device flush with the heart.

Optionally, when blood pressure is measured based on the second blood pressure measurement method, the wearable device may prompt the user to press or touch a side button of the wearable device with one finger.

Optionally, when blood pressure is measured based on the third blood pressure measurement method, the wearable device may prompt the user to keep still, or may not prompt the user to keep still. This is not limited in this application.

Optionally, that the blood pressure is measured based on the second blood pressure measurement method in embodiments of this application may include:
obtaining, by the pressure sensor, a first pressure value on the first button; and prompting, based on the first pressure value, the user to increase or decrease pressing force on the first button.

Specifically, when it is detected that the first pressure value is greater than a first threshold, the user may be prompted to decrease the pressing force on the first button. When it is detected that the first pressure value is less than a second threshold, the user is prompted to increase the pressing force on the first button.

When the blood pressure is measured based on the second blood pressure measurement method, the wearable device may further display a pressure indicator bar, where the pressure indicator bar indicates a pressure value, on the first button, obtained by the pressure sensor.

The wearable device may obtain a second blood pressure value based on the first pressure value, the first heart rate data, and the electrocardiogram data collected by the electrocardiogram sensor. When the pressure value on the first button meets a preset pressure range, the first heart rate data is heart rate data collected by the first heart rate sensor, and the electrocardiogram data is electrocardiogram data collected by the electrocardiogram sensor. For example, the preset pressure range may be from 40 gF to 70 gF.

In some embodiments, the wearable device may alternatively measure the blood pressure based on a plurality of blood pressure measurement methods.

For example, the wearable device may measure the blood pressure based on the first blood pressure measurement method and the second blood pressure measurement method. In a possible implementation, the wearable device may separately prompt the user to keep the wrist wearing the wearable device flush with the heart, and press or touch the side button of the wearable device with one finger. In another possible implementation, the wearable device may prompt the user to simultaneously keep the wrist wearing the wearable device flush with the heart and press or touch the side button of the wearable device with one finger.

For another example, the wearable device may measure the blood pressure based on the first blood pressure measurement method and the third blood pressure measurement method. In a possible implementation, the wearable device may separately prompt the user to keep the wrist wearing the wearable device flush with the heart, and keep still. In another possible implementation, the wearable device may prompt the user to simultaneously keep the wrist wearing the wearable device flush with the heart and keep still.

For another example, the wearable device may measure the blood pressure based on the second blood pressure measurement method and the third blood pressure measurement method. In a possible implementation, the wearable device may separately prompt the user to press or touch the side button of the wearable device with one finger, and keep still. In another possible implementation, the wearable device may prompt the user to simultaneously press or touch the side button of the wearable device with one finger and keep still.

For another example, the wearable device may measure the blood pressure based on the first blood pressure measurement method, the second blood pressure measurement method, and the third blood pressure measurement method. In a possible implementation, the wearable device may separately prompt the user to keep the wrist wearing the wearable device flush with the heart, press or touch the side button of the wearable device with one finger, and keep still. In another possible implementation, the wearable device may prompt the user to simultaneously keep the wrist wearing the wearable device flush with the heart, press or touch the side button of the wearable device with one finger, and keep still.

The plurality of blood pressure measurement methods provided in this application have different accuracy and comfort. For example, accuracy of the first blood pressure measurement method is higher than accuracy of the second blood pressure measurement method, and accuracy of the second blood pressure measurement method is higher than accuracy of the third blood pressure measurement method. Comfort of the first blood pressure measurement method is better than comfort of the second blood pressure measurement method, and comfort of the second blood pressure measurement method is better than comfort of the third blood pressure measurement method.

The first information may be actively input by the user into the wearable device, or may be obtained by the wearable device from another electronic device (for example, a mobile phone) that establishes a connection to the wearable device.

Optionally, first information of different users is different, and the wearable device may recommend different first blood pressure measurement solutions to different users. For a same user, the wearable device may recommend different first blood pressure measurement solutions to different users based on different first information.

In the method, when the wearable device needs to accurately measure the blood pressure of the user, the wearable device may measure the blood pressure based on a blood pressure measurement method with high accuracy. In another time period, the wearable device may measure the blood pressure based on a blood pressure measurement method with better comfort. In other words, the wearable device may use different blood pressure measurement methods at different time, to monitor the blood pressure of the user. This can improve both accuracy of blood pressure measured by the wearable device and comfort of the user during blood pressure measurement.

With reference to the first aspect, in a possible implementation, the first blood pressure measurement solution further includes any one or more of the following: a quantity of times of the first blood pressure measurement method, a quantity of times of the second blood pressure measurement method, a quantity of times of the third blood pressure measurement method, measurement time of the first blood pressure measurement method, measurement time of the second blood pressure measurement method, and measurement time of the third blood pressure measurement method.

The quantity of times of the first blood pressure measurement method, the quantity of times of the second blood pressure measurement method, and the quantity of times of the third blood pressure measurement method are all different, or partially the same, or all the same.

The measurement time of the first blood pressure measurement method, the measurement time of the second blood pressure measurement method, and the measurement time of the third blood pressure measurement method are all different, or partially the same, or all the same.

Optionally, the first blood pressure measurement solution further includes information such as execution time of the first blood pressure measurement solution. The execution time of the first blood pressure measurement solution may be at least 1 day, for example, 1 day, 7 days, or 10 days.

With reference to the first aspect, in a possible implementation, the user information includes one or more of the following: blood pressure, a heart rate, a body temperature, a pulse, a respiratory rate, blood glucose, a smoking history, a drinking history, a history of disease, a medication history, lifestyle habits, gender, age, a height, and a weight. The environment information includes any one or more of the following: time, date, climate, a temperature, altitude, season, and weather.

With reference to the first aspect, in a possible implementation, after the wearable device determines the first blood pressure measurement solution based on the first information, the method further includes: The wearable device prompts the user to view content of the first blood pressure measurement solution.

Optionally, the wearable device may prompt, by using another electronic device (for example, a mobile phone) that establishes a communication connection to the wearable device, the user to view the first blood pressure measurement solution.

Optionally, after receiving the first information input by the user and obtaining the first blood pressure measurement solution based on the first information, the wearable device may prompt the user to view the content of the first blood pressure measurement solution.

Optionally, alternatively, the wearable device may periodically/occasionally recommend the first blood pressure measurement solution to the user, and prompt the user to view the content of the first blood pressure measurement solution.

With reference to the first aspect, in a possible implementation, after the wearable device determines the first blood pressure measurement solution based on the first information, the method further includes: The wearable device obtains a first blood pressure value through measurement, and obtains a second blood pressure measurement solution based on the first blood pressure value.

The second blood pressure measurement solution is the same as or different from the first blood pressure measurement solution.

Optionally, the wearable device may obtain the first blood pressure value before performing the first blood pressure measurement solution or in a process of performing the first blood pressure measurement solution, and determine, based on the first blood pressure value, whether the first blood pressure measurement solution needs to be updated, to obtain the second blood pressure measurement solution.

With reference to the first aspect, in a possible implementation, if the first blood pressure value is high or low, a quantity of execution times of the first blood pressure measurement method in the second blood pressure measurement solution is greater than that of the first blood pressure measurement method in the first blood pressure measurement solution, or a quantity of execution times of the second blood pressure measurement method in the second blood pressure measurement solution is greater than that of the second blood pressure measurement method in the first blood pressure measurement solution.

When the first blood pressure value is high or low, the second blood pressure measurement solution is different from the first blood pressure measurement solution. For example, the quantity of execution times of the first blood pressure measurement method in the second blood pressure measurement solution is greater than the quantity of execution times of the first blood pressure measurement method in the first blood pressure measurement solution, or the quantity of execution times of the second blood pressure measurement method in the second blood pressure measurement solution is greater than the quantity of execution times of the second blood pressure measurement method in the first blood pressure measurement solution. Therefore, when the blood pressure of the user is abnormal, the wearable device may increase the quantity of execution times of the first blood pressure measurement method or the second blood pressure measurement method with high accuracy, to more accurately monitor the blood pressure of the user with abnormal blood pressure.

Optionally, when the quantity of execution times of the first blood pressure measurement method in the second blood pressure measurement solution is greater than the quantity of execution times of the first blood pressure measurement method in the first blood pressure measurement solution, or the quantity of execution times of the second blood pressure measurement method in the second blood pressure measurement solution is greater than the quantity of execution times of the second blood pressure measurement method in the first blood pressure measurement solution, a quantity of execution times of the third blood pressure measurement method in the second blood pressure measurement solution is the same as a quantity of execution times of the third blood pressure measurement method in the first blood pressure measurement solution; or the quantity of execution times of the third blood pressure measurement method in the second blood pressure measurement solution is less than the quantity of execution times of the third blood pressure measurement method in the first blood pressure measurement solution.

With reference to the first aspect, in a possible implementation, if the first blood pressure value is normal, the quantity of execution times of the first blood pressure measurement method in the second blood pressure measurement solution is less than or equal to that of the first blood pressure measurement method in the first blood pressure measurement solution, or the quantity of execution times of the second blood pressure measurement method in the second blood pressure measurement solution is less than or equal to that of the first blood pressure measurement method in the first blood pressure measurement solution.

When the first blood pressure value is normal, the second blood pressure measurement solution is the same as or different from the first blood pressure measurement solution. For example, the quantity of execution times of the first blood pressure measurement method in the second blood pressure measurement solution is less than or equal to the quantity of execution times of the first blood pressure measurement method in the first blood pressure measurement solution, or the quantity of execution times of the second blood pressure measurement method in the second blood pressure measurement solution is less than or equal to the quantity of execution times of the second blood pressure measurement method in the first blood pressure measurement solution. Therefore, when the blood pressure of the user is normal, the wearable device can appropriately reduce a quantity of execution times of blood pressure measurement methods that affect user comfort, to improve user experience.

Optionally, when the quantity of execution times of the first blood pressure measurement method in the second blood pressure measurement solution is less than or equal to the quantity of execution times of the first blood pressure measurement method in the first blood pressure measurement solution, the quantity of execution times of the second blood pressure measurement method in the second blood pressure measurement solution is greater than the quantity of execution times of the second blood pressure measurement method in the first blood pressure measurement solution, the quantity of execution times of the third blood pressure measurement method in the second blood pressure measurement solution is the same as the quantity of execution times of the third blood pressure measurement method in the first blood pressure measurement solution; or the quantity of execution times of the second blood pressure measurement method in the second blood pressure measurement solution is the same as the quantity of execution times of the second blood pressure measurement method in the first blood pressure measurement solution, and the quantity of execution times of the third blood pressure measurement method in the second blood pressure measurement solution is greater than the quantity of execution times of the third blood pressure measurement method in the first blood pressure measurement solution.

Optionally, when the quantity of execution times of the second blood pressure measurement method in the second blood pressure measurement solution is less than or equal to the quantity of execution times of the second blood pressure measurement method in the first blood pressure measurement solution, the quantity of execution times of the first blood pressure measurement method in the second blood pressure measurement solution is less than the quantity of execution times of the first blood pressure measurement method in the first blood pressure measurement solution, and the quantity of execution times of the third blood pressure measurement method in the second blood pressure measurement solution is greater than the quantity of execution times of the third blood pressure measurement method in the first blood pressure measurement solution.

Optionally, when the first blood pressure value is normal, the quantity of execution times of the first blood pressure measurement method in the second blood pressure measurement solution is the same as the quantity of execution times of the first blood pressure measurement method in the first blood pressure measurement solution, the quantity of execution times of the second blood pressure measurement method in the second blood pressure measurement solution is greater than the quantity of execution times of the second blood pressure measurement method in the first blood pressure measurement solution, and the quantity of execution times of the third blood pressure measurement method in the second blood pressure measurement solution is less than the quantity of execution times of the third blood pressure measurement method in the first blood pressure measurement solution.

With reference to the first aspect, in a possible implementation, the first blood pressure value is obtained through measurement based on the first blood pressure measurement method. Therefore, the wearable device may obtain an actual blood pressure value of the user based on the first blood pressure measurement method with highest accuracy, and determine, based on the actual blood pressure value of the user, whether the first blood pressure measurement solution needs to be modified, to improve applicability of obtaining the second blood pressure measurement solution by the wearable device.

With reference to the first aspect, in a possible implementation, after the wearable device determines the first blood pressure measurement solution based on the first information, the method further includes: The wearable device measures blood pressure based on the first blood pressure measurement solution, to obtain a plurality of blood pressure values; and determines a third blood pressure measurement solution based on the plurality of blood pressure values, where the third blood pressure measurement solution is different from the first blood pressure measurement solution. After the wearable device performs the first blood pressure measurement solution for first duration, the wearable device may obtain the plurality of blood pressure values. The wearable device may update the first blood pressure measurement solution based on the plurality of blood pressure values, to obtain the third blood pressure measurement solution. Optionally, the wearable device may alternatively update the first blood pressure measurement solution based on the plurality of blood pressure values and the first information, to obtain the third blood pressure measurement solution.

For example, when determining, based on the plurality of blood pressure values, that the blood pressure of the user is normal, the wearable device 100 may appropriately reduce a quantity of measurement times of the first blood pressure measurement method in the first blood pressure measurement solution or a quantity of measurement times of the second blood pressure measurement method, to improve comfort of blood pressure measurement by the wearable device 100.

For another example, when determining, based on the plurality of blood pressure values, that the blood pressure of the user is abnormal, the wearable device 100 may appropriately increase the quantity of measurement times of the first blood pressure measurement method in the first blood pressure measurement solution or increase the quantity of measurement times of the second blood pressure measurement method. This improves accuracy of blood pressure measured by the wearable device 100, to more accurately monitor and evaluate the blood pressure of the user with abnormal blood pressure.

With reference to the first aspect, in a possible implementation, after the wearable device determines the first blood pressure measurement solution based on the first information, the method further includes: The wearable device obtains the second blood pressure value based on the first blood pressure measurement method; collects the second heart rate data based on the second heart rate sensor; and updates the first target model based on the second blood pressure value and the second heart rate data. A third blood pressure value obtained by the updated first target model based on the second heart rate data is closer to the second blood pressure value than a fourth blood pressure value obtained based on the second heart rate data by the first target model before the update.

That the wearable device updates the first target model based on the second blood pressure value and the second heart rate data may include: The wearable device inputs the second heart rate data to the first target model, and the first target model outputs a predicted blood pressure value. The wearable device may calibrate the first target model based on a difference between the predicted blood pressure value and the second blood pressure value.

In this way, when measuring the blood pressure of the user based on the first blood pressure measurement method, the wearable device may automatically calibrate the first target model in the third blood pressure measurement method based on the blood pressure value obtained based on the first blood pressure measurement method. Therefore, the wearable device can calibrate the first target model without awareness of the user, and can further increase a quantity of times of calibrating the first target model, to help improve accuracy of estimating a blood pressure value by using the first target model.

With reference to the first aspect, in a possible implementation, after the wearable device determines the first blood pressure measurement solution based on the first information, the method further includes: The wearable device obtains the third blood pressure value based on the second blood pressure measurement method; collects the second heart rate data based on the second heart rate sensor; and updates the first target model based on the third blood pressure value and the second heart rate data. A fifth blood pressure value obtained by the updated first target model based on the second heart rate data is closer to the third blood pressure value than a sixth blood pressure value obtained based on the second heart rate data by the first target model before the update.

That the wearable device updates the first target model based on the second blood pressure value and the second heart rate data may include: The wearable device inputs the second heart rate data to the first target model, and the first target model outputs a predicted blood pressure value. The wearable device may calibrate the first target model based on a difference between the predicted blood pressure value and the third blood pressure value.

In this way, when measuring the blood pressure of the user based on the second blood pressure measurement method, the wearable device may automatically calibrate the first target model in the third blood pressure measurement method based on the blood pressure value obtained based on the second blood pressure measurement method. Therefore, the wearable device can calibrate the first target model without awareness of the user, and can further increase a quantity of times of calibrating the first target model, to help improve accuracy of estimating a blood pressure value by using the first target model.

With reference to the first aspect, in a possible implementation, a time interval between first collection time of the second blood pressure value and second collection time of the second heart rate data is less than a first threshold.

In a possible implementation, time interval between first collection time of the third blood pressure value and second collection time of the second heart rate data is also less than the first threshold.

In this way, the time interval between the first collection time of the second blood pressure value or the third blood pressure value and the second collection time of the second heart rate data falls within preset time, for example, 10 minutes or half an hour, to ensure that the second blood pressure value or the third blood pressure value and the second heart rate data are collected when a physical status of the user does not differ greatly. This can improve accuracy of calibrating the first target model based on the second blood pressure value or the third blood pressure value by the wearable device.

Optionally, after the first blood pressure measurement solution is executed, the wearable device 100 may provide, based on the data obtained through measurement and the blood pressure results, a comprehensive blood pressure evaluation result of the user that includes but not limited to a maximum value, a minimum value, and an average value of systolic pressure and diastolic pressure during measurement, a blood pressure change trend diagram formed based on the measurement results, a valid data proportion, and other analysis results; and provide proper suggestions for the user, for example, an exercise suggestion or a rest suggestion.

According to a second aspect, this application provides a wearable device. The wearable device includes a memory and a processor. The memory is coupled to the processor, the memory is configured to store a computer program, and when the processor invokes the computer program, the wearable device is enabled to perform the blood pressure measurement method provided in any possible implementation of any one of the foregoing aspects.

According to a third aspect, this application provides a computer-readable storage medium, including instructions. When the instructions are run on a wearable device, the wearable device is enabled to perform the blood pressure measurement method provided in any possible implementation of any one of the foregoing aspects.

According to a fourth aspect, this application provides a chip system. The chip system includes one or more processors, and the processor is configured to invoke computer instructions, to enable a wearable device to perform the blood pressure measurement method provided in any possible implementation of any one of the foregoing aspects.

According to a fifth aspect, this application provides a computer program product including instructions. When the computer program product runs on a wearable device, the wearable device is enabled to perform the blood pressure measurement method provided in any possible implementation of any one of the foregoing aspects.

For descriptions of beneficial effect of the second aspect to the fifth aspect, refer to the descriptions of the beneficial effect of the first aspect. Details are not described again in this application.

### BRIEF DESCRIPTION OF DRAWINGS

FIG. 1 and FIG. 2 are diagrams of a structure of a wearable device 100;
FIG. 3A to FIG. 3D are diagrams of several blood pressure measurement postures;
FIG. 4 is a diagram of a hardware structure of a wearable device 100;
FIG. 5 is a schematic flowchart of a method in which a wearable device 100 determines a blood pressure measurement solution;
FIG. 6A to FIG. 6C are diagrams in which a wearable device 100 prompts a user to view, modify, and save a first blood pressure measurement solution;
FIG. 7A is a schematic flowchart of a method in which a wearable device 10 determines, based on a blood pressure value of a user, whether to modify a first blood pressure measurement solution;
FIG. 7B is a diagram in which a wearable device 100 prompts a user to view an updated blood pressure measurement solution;
FIG. 8A to FIG. 8E are diagrams in which a wearable device 100 initially calibrates a second target model;
FIG. 8F is a diagram in which a wearable device 100 initially calibrates a first target model;
FIG. 8G is a diagram in which a wearable device 100 prompts a user to measure blood pressure based on a first blood pressure measurement method;
FIG. 8H is a schematic flowchart of a method in which a wearable device 100 obtains a blood pressure value based on a first blood pressure measurement method;
FIG. 8I is a diagram in which a wearable device 100 prompts a user to measure blood pressure based on a second blood pressure measurement method;
FIG. 8J is a diagram in which a wearable device 100 prompts a user to measure blood pressure based on a third blood pressure measurement method;
FIG. 9A to FIG. 9C are diagrams in which a wearable device 100 performs non-initial calibration on a blood pressure model;
FIG. 9D to FIG. 9G are diagrams in which a wearable device 100 measures blood pressure based on a plurality of blood pressure measurement methods;
FIG. 10A(a) to FIG. 10A(c) and FIG. 10B are diagrams of impact of pressure on a PPG signal and an ECG signal;
FIG. 11 is a schematic flowchart of a method in which a wearable device 100 monitors a magnitude of pressure and adjusts the magnitude of the pressure;
FIG. 12A to FIG. 12C are diagrams in which a wearable device 100 prompts a user to adjust pressing force;
FIG. 13A and FIG. 13B are a schematic flowchart of a method in which a wearable device 100 monitors an amplitude of a PPG signal and adjusts a magnitude of pressure;
FIG. 14A and FIG. 14B are diagrams in which another wearable device 100 prompts a user to adjust pressing force; and
FIG. 15A and FIG. 15B are diagrams in which a wearable device 100 prompts a user to keep still.

### DESCRIPTION OF EMBODIMENTS

The technical solutions according to embodiments of this application are clearly and completely described in the following with reference to accompanying drawings. In the descriptions of embodiments of this application, unless otherwise specified, "/" indicates "or". For example, A/B may indicate A or B. The term "and/or" in this specification merely describes an association relationship between associated objects, and indicates that three relationships may exist. For example, A and/or B may indicate the following three cases: Only A exists, both A and B exist, or only B exists. In addition, in the descriptions of embodiments of this application, "a plurality of" means two or more.

In the following descriptions, the terms "first" and "second" are merely intended for a purpose of description, and shall not be interpreted as an implication or implication of relative importance or implicit indication of a quantity of indicated technical features. Therefore, a feature limited by "first" and "second" may explicitly or implicitly include one or more features. In the descriptions of embodiments of this application, unless otherwise specified, "a plurality of" means two or more.

A term "user interface (user interface, UI)" in the following embodiments of this application is a medium interface for interaction and information exchange between an application or an operating system and a user, and implements conversion between an internal form of information and a form acceptable to the user. The user interface is usually represented in a form of a graphical user interface (graphical user interface, GUI), which is a user interface displayed in a graphical manner and related to computer operations.

To facilitate blood pressure measurement of users, wearable devices already have blood pressure measurement components, and the users may measure blood pressure by using the wearable devices 100 worn by the users.

The wearable device 100 may provide a plurality of blood pressure measurement components, and different blood pressure measurement components measure blood pressure with different accuracy and comfort.

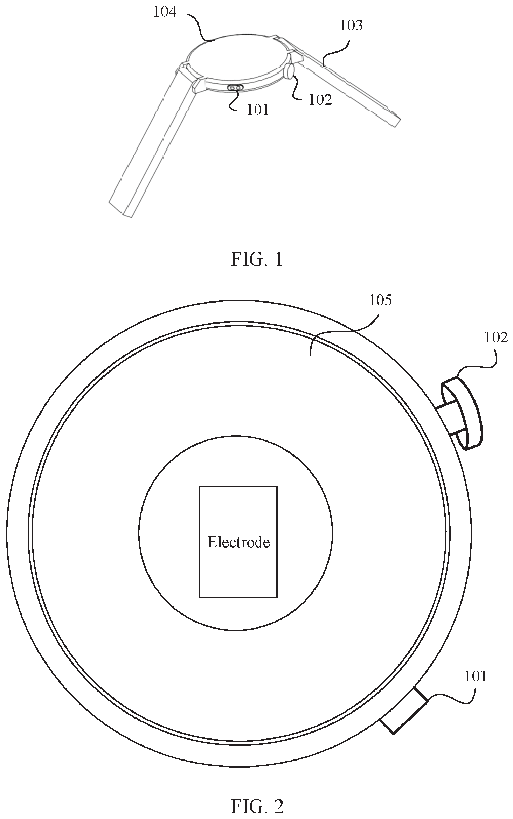

FIG. 1 and FIG. 2 are diagrams of a structure of a wearable device 100.

As shown in FIG. 1, the wearable device 100 includes a watch body and a wearable component 103, and the watch body includes a case back (not shown in FIG. 1) and a display surface 104.

The display surface 104 includes a display screen. The display screen may be configured to display time, a battery level, a Bluetooth identifier, a received message, motion data of a user, and other content. The display screen may be configured to receive a user tap operation to light the display screen, and the like. The display screen may further record a quantity of moving steps and consumed energy of the user, and has basic functions such as an incoming call reminder and a message notification.

The wearable component 103 is configured to mount the watch body. For example, the wearable component 103 may be an apparatus like a strap or a bracelet. The wearable component 103 is an apparatus that can attach the watch body to the wrist of the user. The wearable device 100 is attached to the wrist of the user.

A side face of the watch body includes a button 101 and a button 102.

In some embodiments, a first heart rate sensor is disposed on the button 101, and the first heart rate sensor is configured to monitor first heart rate data in contact with the button 101. When blood pressure needs to be measured, the user may press or touch the button 101 with one finger, and the wearable device 100 may collect the first heart rate data of the finger of the user by using the first heart rate sensor on the button 101. The wearable device 100 may obtain a blood pressure value of the user based on the first heart rate data.

Optionally, a pressure sensor may be further disposed on the button 101. The pressure sensor is configured to monitor a pressure signal in contact with the button 101. When the user presses or touches the button 101 with one finger, the wearable device 100 may collect pressure data of the finger of the user by using the pressure sensor on the button 101. The wearable device 100 may obtain the blood pressure value of the user based on the pressure data and the first heart rate data.

Optionally, an electrode like a first electrode may be further disposed on a surface of the button 101. When the blood pressure needs to be measured, the user may press the button 101 with one finger, the first electrode on the surface of the button 101 is in contact with finger skin, and an electrode (for example, the second electrode) on the case back is in contact with wrist skin, so that the first electrode and the second electrode form a loop, and the wearable device 100 may collect electrocardiogram data by using the first electrode and the second electrode. The wearable device 100 may estimate the blood pressure value of the user based on the first heart rate data and the electrocardiogram data, or the first heart rate data, the electrocardiogram data, and the pressure data. In some embodiments, the first electrode and the second electrode may be referred to as electrocardiogram sensors. The electrocardiogram sensors may further include more other electrodes. For example, the case back 105 may further include a third electrode, and the wearable device 100 may collect an ECG signal by using the first electrode, the second electrode, and the third electrode. Optionally, in addition to the first electrode, more other electrodes may also be disposed on the button 101. In addition to electrodes disposed on the button 101, one or more electrodes may be further disposed at other positions of the watch body for the wearable device 100, for example, one or more electrodes are disposed on the button 102. The button 102 may be configured to control the display screen to be on or off, scroll, based on a user operation, a page displayed in the display screen, and the like.

In some embodiments, the wearable device 100 may include components such as an inflatable component, a bulb, and a barometric pressure sensor (not shown in FIG. 1). When the blood pressure needs to be measured, the inflatable component in the wearable device 100 may inflate the bulb with increasing pressure, and block an arterial blood flow via atmospheric pressure. The barometric pressure sensor may obtain oscillation waves of different pressure, to determine information such as systolic pressure and diastolic pressure.

It may be understood that the wearable device may alternatively not include the bulb.

FIG. 2 is a diagram of a case back of a wearable device 100.

As shown in FIG. 2, the case back 105 of the watch body includes the second electrode, and the second electrode of the case back 105 is configured to form the loop with the first electrode on the surface of the button 101 to collect the electrocardiogram data.

In some embodiments, a second heart rate sensor is disposed on the case back 105, and the second heart rate sensor is configured to monitor second heart rate data in contact with the button 101. When the blood pressure needs to be measured, the wearable device 100 may collect the second heart rate data by using the second heart rate sensor disposed on the case back 105. The wearable device 100 may determine the blood pressure value of the user based on the second heart rate data.

In some embodiments, a pressure sensor may also be disposed on the case back 105, and the pressure sensor is configured to monitor a pressure signal in contact with the case back 105. When the blood pressure needs to be measured, the wearable device 100 may collect pressure data of the wrist by using the pressure sensor disposed on the case back 105. The wearable device 100 may determine the blood pressure value of the user based on the second heart rate data and the pressure data of the wrist.

Optionally, the first heart rate sensor or the second heart rate sensor may be any one of a photoplethysmography (photoplethysmography, PPG) sensor, a laser sensor, an ultrasonic sensor, and a magnetic induction sensor. In embodiments of this application, an example in which the first heart rate sensor or the second heart rate sensor is a PPG sensor, and the first heart rate data or the second heart rate data is a PPG signal is used for description.

Optionally, the PPG sensor may include a light-emitting source and a photoelectric detector. The light-emitting source may emit red light, or may emit infrared light, or may emit other light. There may be one or more light-emitting sources. For example, the light-emitting source may be a light-emitting apparatus like a light-emitting diode (light-emitting diode, LED). The photoelectric detector is configured to receive light emitted by the light-emitting source. There may be one or more photoelectric detectors. For example, the photoelectric detector may be a receiving apparatus like a photodiode (photodiode, PD).

The PPG signal may include but is not limited to a pulse wave signal, and may further include another signal. This is not limited in this application.

Optionally, the electrocardiogram sensor may be an electrocardiogram (electrocardiogram, ECG) sensor, and the electrocardiogram data may be an ECG signal.

Optionally, FIG. 1 and FIG. 2 show only a form of the wearable device 100. The wearable device 100 may alternatively be a device in another form, for example, a device that does not include a display screen, for example, a ring or a band. A sensor may also be disposed on a device body for the device in the another form. The device in the another form may also include one or more sensors, and measure blood pressure based on sensor data collected by the one or more sensors.

Based on analysis in FIG. 1 and FIG. 2, the wearable device 100 may provide a plurality of different blood pressure measurement methods, and different blood pressure measurement methods may be completed by using different blood pressure measurement components.

For example, the wearable device 100 may include a first blood pressure measurement method. The first blood pressure measurement method may be implemented by using a first blood pressure measurement component. The first blood pressure measurement component may include but is not limited to components such as the inflatable component, the bulb, and the barometric pressure sensor.

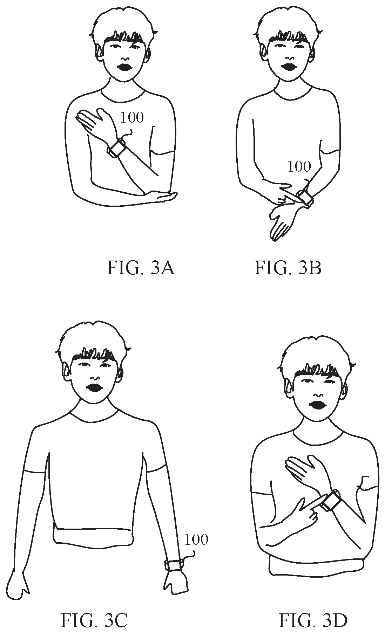

Optionally, when the blood pressure is measured based on the first blood pressure measurement method, the user may maintain a blood pressure measurement posture shown in FIG. 3A, that is, keep the wrist wearing the wearable device 100 flush with the heart.

For another example, the wearable device 100 may include a second blood pressure measurement method. The second blood pressure measurement method may be implemented by using a second blood pressure measurement component. The second blood pressure measurement component may include the first heart rate sensor, or the first heart rate sensor and the pressure sensor, or the first heart rate sensor, the electrocardiogram sensor, and the pressure sensor. The pressure sensor is configured to monitor a pressure signal in contact with the button 101.

Optionally, when the blood pressure is measured based on the second blood pressure measurement method, the user may maintain a blood pressure measurement posture shown in FIG. 3B, that is, a finger of the user presses the button 101.

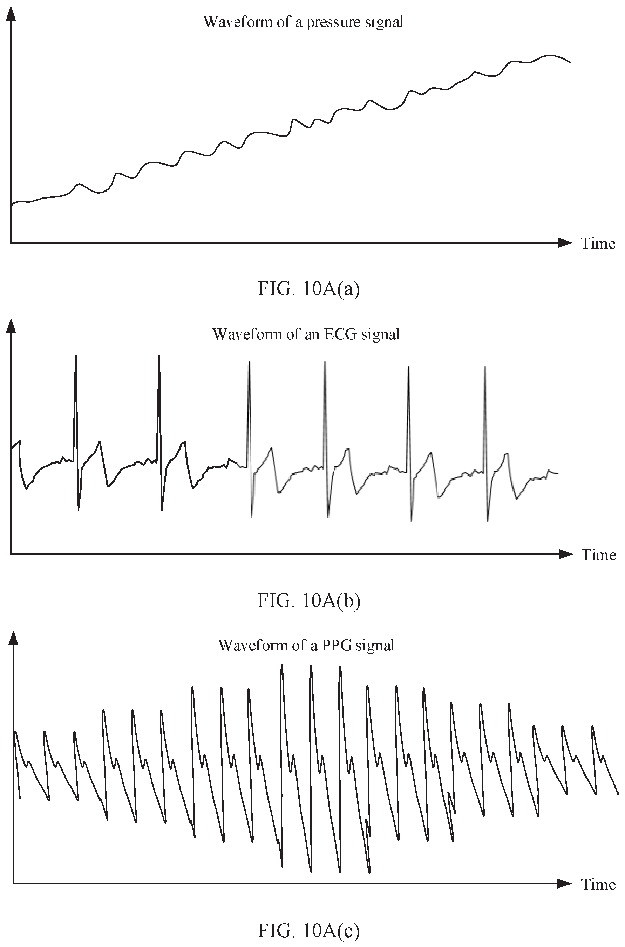

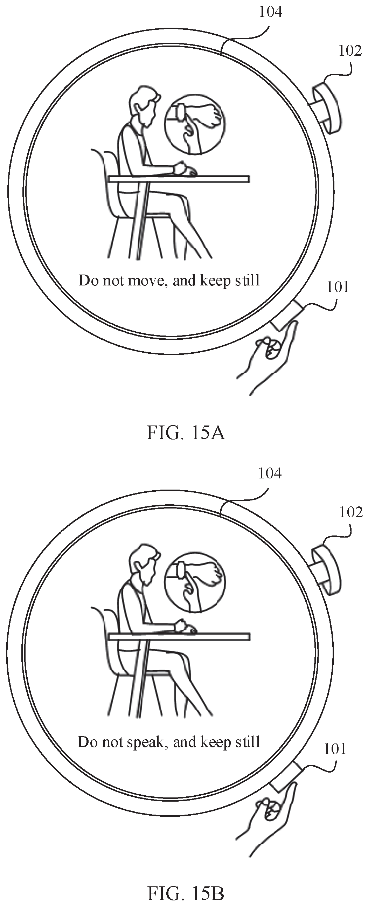

Optionally, a method shown in FIG. 10A(a) to FIG. 10A(c) to FIG. 15B may be used to implement blood pressure measurement based on the second blood pressure measurement method.

Based on the foregoing descriptions, the wearable device 100 may estimate the blood pressure value of the user based on sensor data collected by the preset sensors. To improve accuracy of the estimated blood pressure value, reliability and accuracy of the sensor data collected by the wearable device 100 need to be ensured.

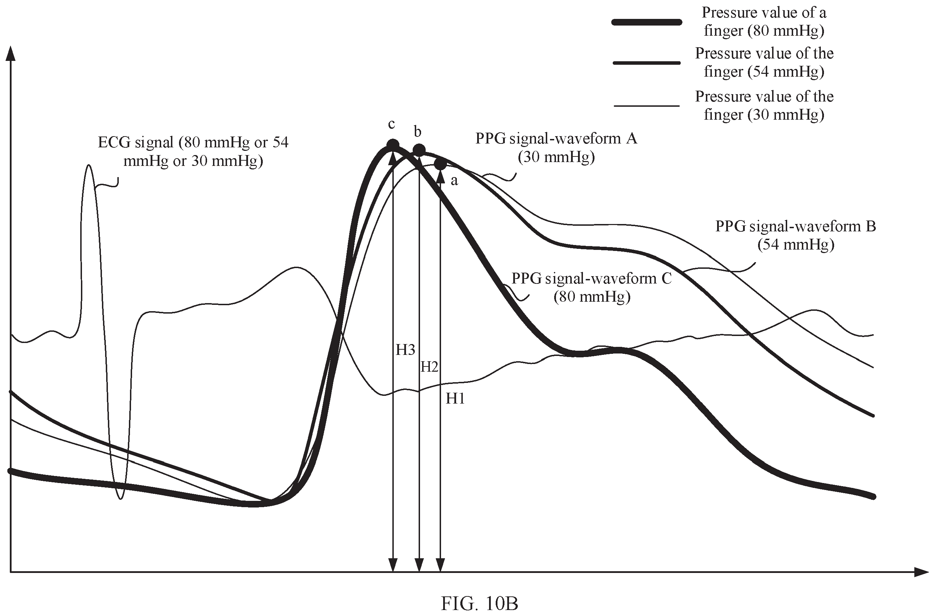

FIG. 10A(a) to FIG. 10A(c) and FIG. 10B are diagrams of impact of pressure on a PPG signal and an ECG signal.

FIG. 10A(a) shows a diagram of a waveform of a pressure signal, FIG. 10A(b) shows a diagram of a waveform of the ECG signal under different pressure, and FIG. 10A(c) shows a diagram of a waveform of the PPG signal under different pressure.

It can be learned from FIG. 10A(a) and FIG. 10A(b) that, as the pressure gradually increases, an amplitude of the ECG signal does not fluctuate obviously. It indicates that the pressure has small impact on the ECG signal.

It can be learned from FIG. 10A(a) and FIG. 10A(c) that, as the pressure gradually increases, an amplitude of the PPG signal first increases and then decreases. It indicates that the pressure has obvious impact on the PPG signal.

FIG. 10B is a diagram of waveforms of the ECG signal and the PPG signal under different pressure.

As shown in FIG. 10B, under pressure of: 30 mmHg, 54 mmHg, and 80 mmHg, waveforms of the ECG signal do not change obviously, and the waveforms of the ECG signal are basically the same.

Under the pressures of: 30 mmHg, 54 mmHg, and 80 mmHg, waveforms of the PPG signal are different. As shown in FIG. 10B, a waveform of the PPG signal is a waveform A under the pressure of 30 mmHg, the waveform A includes a feature point a, the feature point a represents a position at which central arterial pressure is the largest, and a maximum value of the central arterial pressure is H1. A waveform of the PPG signal is a waveform B under the pressure of 54 mmHg, the waveform B includes a feature point b, the feature point b also represents a position at which central arterial pressure is the largest, and a maximum value of the central arterial pressure is H2. A waveform of the PPG signal is a waveform C under the pressure of 80 mmHg, the waveform C includes a feature point c, the feature point c also represents a position at which central arterial pressure is the largest, and a maximum value of the central arterial pressure is H3.

In some embodiments, the maximum value of the central arterial pressure may also be referred to as the amplitude of the PPG signal.

H1 is less than H2, and H2 is less than H3. In other words, the maximum value of the central arterial pressure of the PPG signal varies with the pressure. In other words, the amplitude of the PPG signal also varies with the pressure.

FIG. 10B only shows that as the pressure increases, the amplitude of the PPG signal gradually increases. After the pressure exceeds a preset value, it can be learned from FIG. 10A(a) to FIG. 10A(c) that, as the pressure continues to increase, the amplitude of the PPG signal gradually decreases.

It can be learned from analysis in FIG. 10A(a) to FIG. 10A(c) and FIG. 10B that the pressure affects the amplitude of the PPG signal. As the pressure increases, the amplitude of the PPG signal first increases and then decreases. The pressure has little impact on the amplitude of the PPG signal. Therefore, the wearable device 100 may prompt, based on the pressure value obtained by the pressure sensor, the user to adjust pressing force, to change the amplitude of the PPG signal until the amplitude of the PPG signal is greater than a preset amplitude.

To improve accuracy of estimating the blood pressure value of the user by the wearable device 100, quality of the PPG signal and quality of the ECG signal need to be monitored, and the quality of the PPG signal and the ECG signal that are collected by the wearable device 100 needs to be improved.

**The wearable device 100 may monitor the quality of the PPG signal.**

The quality of the PPG signal may be related to the amplitude of the PPG signal and stability of the PPG signal. When the amplitude of the PPG signal is greater than the preset amplitude, and the PPG signal is stable, the PPG signal has a strong anti-noise interference capability, and a blood pressure value obtained by the wearable device 100 through estimation based on a plurality of parameters such as the PPG signal is closer to an actual blood pressure value of the user.

**First, how the wearable device 100 monitors the amplitude of the PPG signal is described.**
**1. The wearable device 100 monitors the amplitude of the PPG signal.**

Based on the descriptions of embodiments in FIG. 10A(a) to FIG. 10A(c) and FIG. 10B, the amplitude of the PPG signal is related to the magnitude of the pressure. As the pressure gradually increases, the amplitude of the PPG signal first increases and then decreases. In this case, within a preset pressure range, the amplitude of the PPG signal is greater than the preset amplitude. The preset pressure range may mean that the pressure value is between a first threshold and a second threshold, and the second threshold is greater than the first threshold.

**In some embodiments, the wearable device 100 may monitor the pressure value, and adjust the pressure value to the preset pressure range, to obtain a PPG signal with a high amplitude.**

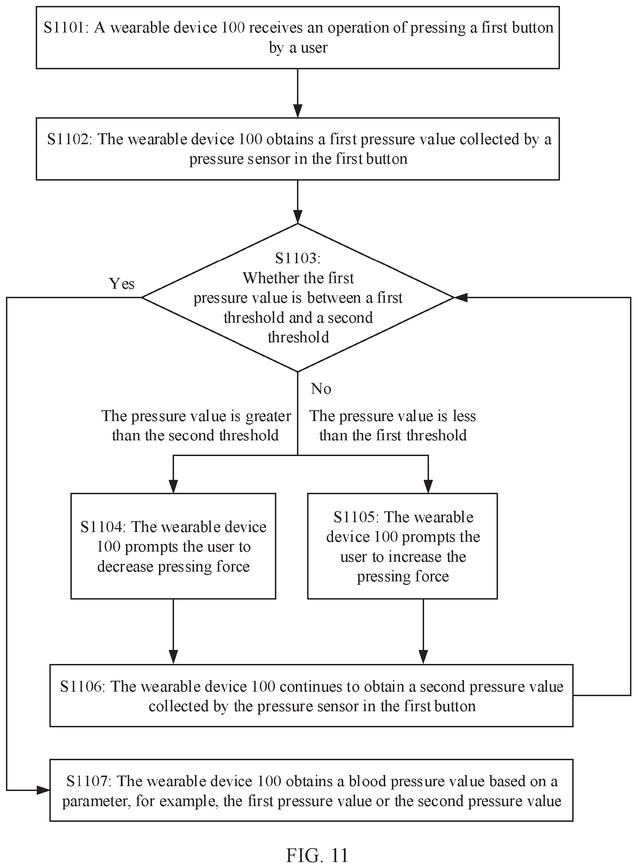

FIG. 11 is a schematic flowchart of a method in which a wearable device 100 monitors a magnitude of pressure and adjusts the magnitude of the pressure.

S1001: The wearable device 100 receives an operation of pressing a first button by a user.

In some embodiments, the button 101 is disposed on a side of the wearable device 100, and the button 101 may also be referred to as the first button. When the user presses the button 101 with one finger, the wearable device 100 may collect pressing force of the finger by using the pressure sensor preset in the button 101, and collect a PPG signal of the finger by using a PPG sensor on the button 101.

S1102: The wearable device 100 obtains a first pressure value collected by the pressure sensor in the first button.

When the user presses the first button with one finger, the wearable device 100 may obtain the first pressure value collected by the pressure sensor in the first button.

S1103: The wearable device 100 needs to determine whether the first pressure value is between a first threshold and a second threshold.

After obtaining the first pressure value collected by the pressure sensor, the wearable device 100 needs to determine whether the pressure value falls within the preset pressure range. The preset pressure range may mean that the pressure value is between the first threshold and the second threshold, and the second threshold is greater than the first threshold.

When the first pressure value is between the first threshold and the second threshold, it indicates that the pressing force of the user is proper, and magnitude of the pressing force does not need to be adjusted, and S1107 is performed.

When the first pressure value is not between the first threshold and the second threshold, it indicates that the pressing force of the user is too large or too small, and the magnitude of the pressing force needs to be adjusted, so that the pressing force is between the first threshold and the second threshold, and S1104 or S1105 is performed.

S1104: When the pressure value is greater than the second threshold, the wearable device 100 prompts the user to decrease the pressing force.

S1105: When the pressure value is less than the first threshold, the wearable device 100 prompts the user to increase the pressing force.

When determining that the first pressure value is not between the first threshold and the second threshold, the wearable device 100 needs to further determine that the first pressure value is greater than the second threshold or less than the first threshold.

When the first pressure value is greater than the second threshold, it indicates that the pressing force is too large, and the wearable device 100 may prompt the user to decrease the pressing force.

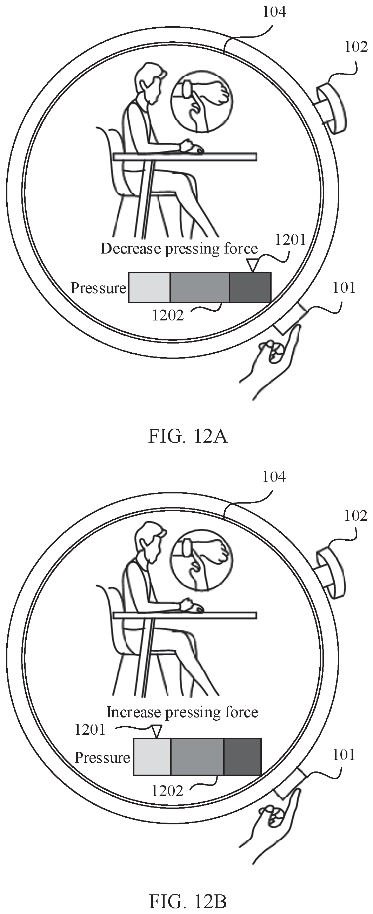

For example, as shown in FIG. 12A, when the first pressure value is greater than the second threshold, the wearable device 100 may display a user interface shown in FIG. 12A. The user interface shown in FIG. 12A includes prompt information "Decrease pressing force", and the prompt information indicates that the pressing force of the user is too large, and the pressing force needs to be decreased.

The user interface shown in FIG. 12A further includes a pressure indication bar 1201. The pressure indication bar 1201 indicates the first pressure value. For example, the first pressure value may be 150 mmHg.

The user interface shown in FIG. 12A may further include a preset pressure range 1202. Optionally, the preset pressure range 1202 may include the first threshold and the second threshold. For example, the first threshold is 60 mmHg, and the second threshold is 140 mmHg.

For another example, the preset pressure range 1202 may be from 40 gF to 70 gF, that is, the first threshold may be 40 gF, and the second threshold may be 70 gF.

When the first pressure value is less than the first threshold, it indicates that the pressing force is too small, and the wearable device 100 may prompt the user to increase the pressing force.

For example, as shown in FIG. 12B, when the first pressure value is less than the first threshold, the wearable device 100 may display a user interface shown in FIG. 12B. The user interface shown in FIG. 12B includes prompt information "Increase pressing force", and the prompt information indicates that the pressing force of the user is too small, and the pressing force needs to be increased.

The user interface shown in FIG. 12B also includes the pressure indication bar 1201. The pressure indication bar 1201 indicates the first pressure value. For example, the first pressure value may be 50 mmHg.

The user interface shown in FIG. 12B may also include the preset pressure range 1202.

S1106: The wearable device 100 continues to obtain a second pressure value collected by the pressure sensor in the first button.

After the wearable device 100 prompts the user to increase or decrease the pressing force, the user may correspondingly adjust the magnitude of the pressing force. The wearable device 100 may continue to obtain the second pressure value collected by the pressure sensor in the first button.

After obtaining the second pressure value, the wearable device 100 needs to continue to monitor whether the second pressure value is between the first threshold and the second threshold. In other words, S1102 is performed until the second pressure value collected by the pressure sensor in the first button is greater than the first threshold and less than the second threshold.

Optionally, when the second pressure value is greater than the first threshold and less than the first threshold, the wearable device 100 may prompt the user that the pressing force is proper, and the current pressing force needs to be maintained.

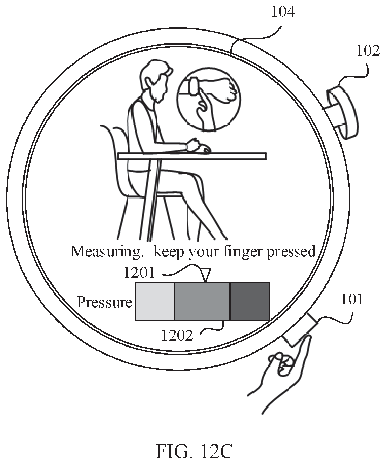

For example, as shown in FIG. 12C, when the second pressure value is greater than the first threshold and less than the first threshold, the wearable device 100 may display a user interface shown in FIG. 12C. The user interface shown in FIG. 12C includes prompt information "Measuring...keep your finger pressed", and the prompt information indicates that the pressing force is proper, and the current pressing force needs to be maintained.

The user interface shown in FIG. 12C also includes the pressure indication bar 1201. The pressure indication bar 1201 indicates the second pressure value. For example, the second pressure value may be 90 mmHg.

The user interface shown in FIG. 12C may also include the preset pressure range 1202.

S1107: The wearable device 100 obtains a blood pressure value based on a parameter, for example, the first pressure value or the second pressure value.

When the first pressure value is greater than the first threshold and less than the second threshold, or the second pressure value is greater than the first threshold and less than the second threshold, the wearable device 100 may obtain the blood pressure value of the user through estimation based on the parameter, for example, the first pressure value or the second pressure value. The blood pressure value of the user includes but is not limited to systolic pressure, diastolic pressure, average blood pressure, and the like.

In other words, the pressure value of the user on the first button may be maintained within a specific preset pressure range based on the foregoing prompt.

It may be understood that, for the preset pressure range described in embodiments of this application, a person skilled in the art may adaptively adjust the preset range based on different units of force, different wearable devices, and different buttons, for example, adjust the first threshold and/or the second threshold.

**In some embodiments, alternatively, the wearable device 100 may directly monitor the amplitude of the PPG signal, and indicate, based on the amplitude of the PPG signal, the user to increase or decrease the pressing force, so that the PPG signal amplitude is greater than a preset value.**

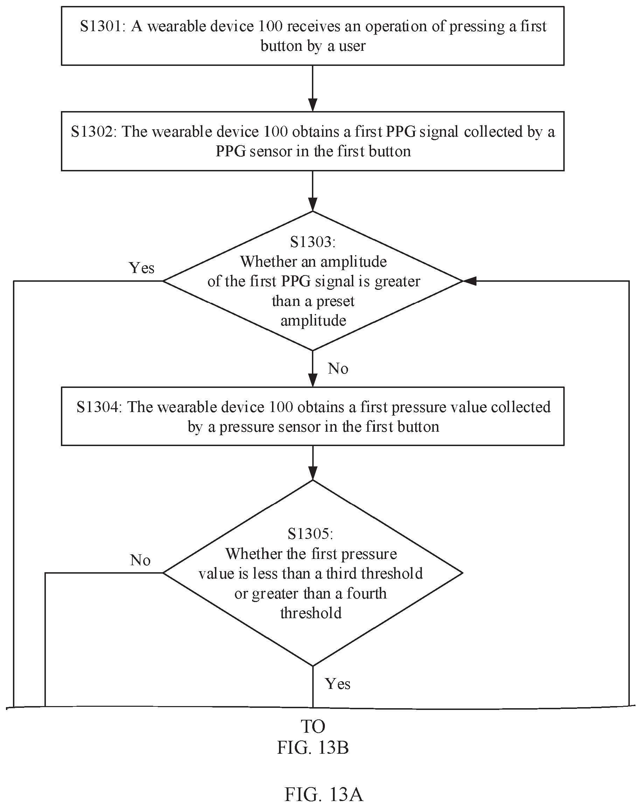

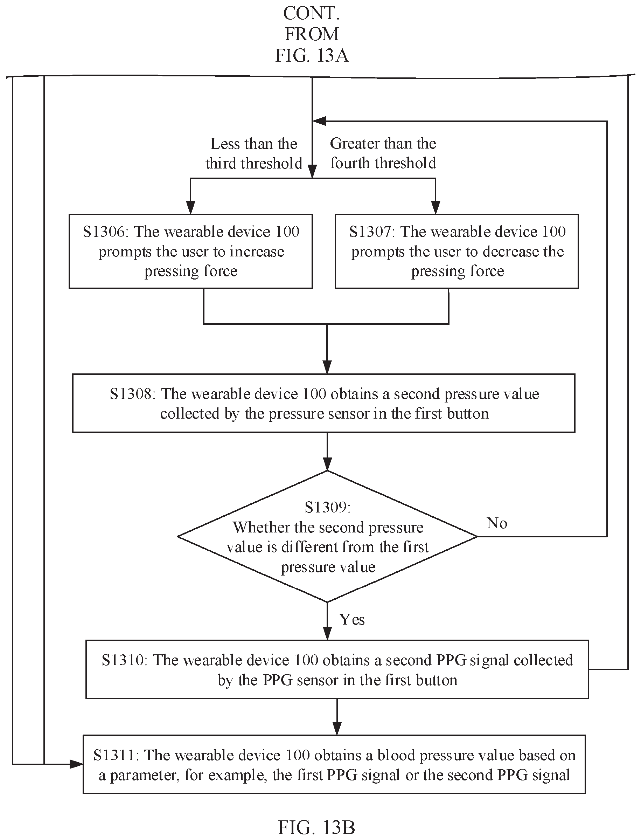

**FIG. 13A** **and** **FIG. 13B** **are a schematic flowchart of a method in which a wearable device 100 monitors an amplitude of a PPG signal and adjusts a magnitude of pressure.**

S1301: The wearable device 100 receives an operation of pressing a first button by a user.

In some embodiments, the button 101 is disposed on a side of the wearable device 100, and the button 101 may also be referred to as the first button. When the user presses the button 101 with one finger, the wearable device 100 may collect a pressure signal of the finger by using the pressure sensor preset in the button 101, and collect a PPG signal of the finger by using a PPG sensor on the button 101.

S1302: The wearable device 100 obtains a first PPG signal collected by the PPG sensor in the first button. It may be understood that the first PPG signal may be first heart rate data.

When the user presses the first button with one finger, the wearable device 100 may obtain the first PPG signal collected by the PPG sensor in the first button.

S1303: The wearable device 100 needs to determine whether an amplitude of the first PPG signal is greater than a preset amplitude.

After obtaining the first PPG signal collected by the PPG sensor, the wearable device 100 needs to determine whether the amplitude of the first PPG signal is greater than the preset amplitude.

When the amplitude of the first PPG signal is greater than the preset amplitude, it indicates that quality of the PPG signal is good, and the amplitude of the PPG signal does not need to be adjusted, and S1311 is performed.

When the amplitude of the first PPG signal is less than the preset amplitude, it indicates that quality of the PPG signal is poor, and magnitude of pressing force needs to be adjusted, so that the amplitude of the PPG signal is greater than the preset amplitude, and S1304 is performed.

S1304: The wearable device 100 obtains a first pressure value collected by the pressure sensor in the first button.

When determining that the amplitude of the first PPG signal is less than the preset amplitude, the wearable device 100 needs to obtain the first pressure value collected by the pressure sensor in the first button. In addition, the pressing force of the user on the button 101 is adjusted based on the first pressure value, to increase the amplitude of the PPG signal, and obtain a PPG signal with good quality.

S1305: The wearable device 100 determines whether the first pressure value is less than a third threshold or greater than a fourth threshold.

When the first pressure value is less than the third threshold or greater than the fourth threshold, S1306 or S1307 is performed.

When the first pressure value is greater than the third threshold and less than the fourth threshold, S1311 is performed.

S1306: When the first pressure value is less than the third threshold, the wearable device 100 prompts the user to increase the pressing force.

S1307: When the first pressure value is greater than the fourth threshold, the wearable device 100 prompts the user to decrease the pressing force.

In some embodiments, the third threshold is less than the fourth threshold, the third threshold may be the same as a first threshold, and the fourth threshold may be the same as a second threshold.

In some embodiments, the third threshold is less than the fourth threshold, the third threshold is greater than the first threshold, and the fourth threshold is less than the second threshold.

After determining that the amplitude of the first PPG signal is less than the preset amplitude, and obtaining the first pressure value collected by the pressure sensor, the wearable device 100 may prompt, based on a magnitude of the first pressure value, the user to increase or decrease the pressing force.

When the first pressure value is less than the third threshold, it indicates that the amplitude of the PPG signal is less than the preset amplitude because the pressing force is too small, and the wearable device 100 may prompt the user to increase the pressing force.

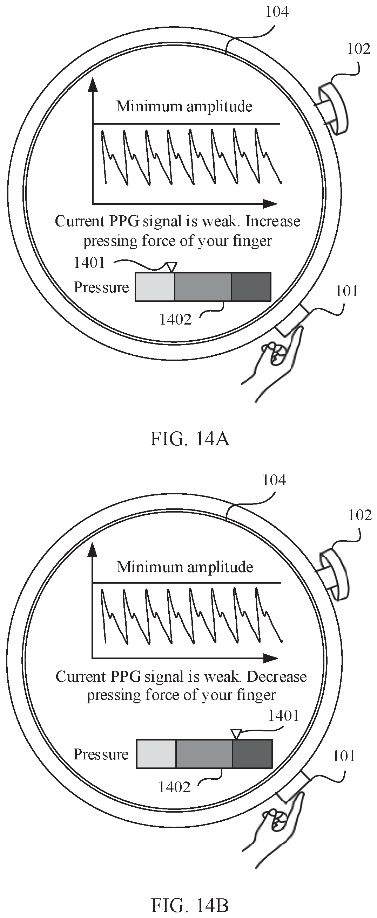

For example, as shown in FIG. 14A, when the first pressure value is less than the third threshold, the wearable device 100 may display a user interface shown in FIG. 14A. The user interface shown in FIG. 14A includes prompt information "Current PPG signal is weak. Increase pressing force of your finger", and the prompt information indicates that the pressing force of the user is too small, and the pressing force needs to be increased.

The user interface shown in FIG. 14A further includes a pressure indication bar 1401. The pressure indication bar 1401 indicates the first pressure value. For example, the first pressure value may be 70 mmHg.

The user interface shown in FIG. 14A may further include a preset pressure range 1402, and the preset pressure range 1402 may be a pressure range indicated by a second square shown in FIG. 14A. Optionally, the preset pressure range 1402 may include the third threshold and the fourth threshold. For example, the third threshold is 80 mmHg, and the fourth threshold is 130 mmHg. For example, a pressure value indicated by the left edge of the second square shown in FIG. 14A may be the third threshold, and a pressure value indicated by the right edge of the second square shown in FIG. 14A may be the fourth threshold.

It may be understood that, for the preset pressure range described in embodiments of this application, a person skilled in the art may adaptively adjust the preset range based on different units of force, different wearable devices, and different buttons, for example, adjust the third threshold and/or the fourth threshold.

When the first pressure value is greater than the fourth threshold, it indicates that the amplitude of the PPG signal is less than the preset amplitude because the pressing force is too large, and the wearable device 100 may prompt the user to decrease the pressing force.

For example, as shown in FIG. 14B, when the first pressure value is greater than the fourth threshold, the wearable device 100 may display a user interface shown in FIG. 14B. The user interface shown in FIG. 14B includes prompt information "Current PPG signal is weak. Decrease pressing force of your finger", and the prompt information indicates that the pressing force of the user is too large, and the pressing force needs to be decreased.

The user interface shown in FIG. 14B also includes the pressure indication bar 1401. The pressure indication bar 1401 indicates the first pressure value. For example, the first pressure value may be 140 mmHg.

The user interface shown in FIG. 14B may also include the preset pressure range 1402.

S1308: The wearable device 100 continues to obtain a second pressure value collected by the pressure sensor in the first button.

After the wearable device 100 prompts the user to increase or decrease the pressing force, the user may correspondingly adjust the magnitude of the pressing force. The wearable device 100 may continue to obtain the second pressure value collected by the pressure sensor in the first button.

S1309: The wearable device 100 needs to determine whether the second pressure value is different from the first pressure value.

After obtaining the second pressure value collected by the pressure sensor, the wearable device 100 needs to determine whether the second pressure value is different from the first pressure value, to determine whether the user adjusts the pressing force.

When the second pressure value is different from the first pressure value, it indicates that the user adjusts the pressing force, and S1310 is performed.

When the second pressure value is the same as the first pressure value, it indicates that the user does not adjust the pressing force, and S1306 or S1307 continues to be performed until the second pressure value is different from the first pressure value.

It may be understood that, a condition may be added, that is, it needs to be determined that the second pressure value is different from the first pressure value and that the second pressure value falls within the preset pressure range. In other words, when the second pressure value is different from the first pressure value, and meets the preset range, S1310 is performed.

When the second pressure value is the same as the first pressure value or the second pressure value does not meet the preset pressure range, it indicates that the user does not adjust the pressing force or even if the pressure is adjusted, the preset pressure range is not met, and S1306 or S1307 continues to be performed until the second pressure value is different from the first pressure value and meets the preset pressure range.

S1310: The wearable device 100 obtains a second PPG signal collected by the PPG sensor in the first button. It may be understood that the second PPG signal may be the first heart rate data.

After the user adjusts the pressing force, the wearable device 100 may obtain the second PPG signal collected by the PPG sensor in the first button, and determine whether an amplitude of the second PPG signal is greater than the preset amplitude, that is, continue to perform S1303.

S1311: The wearable device 100 obtains a blood pressure value based on a parameter, for example, the first PPG signal or the second PPG signal.

When the amplitude of the first PPG signal is greater than the preset amplitude, or the amplitude of the second PPG signal is greater than the preset amplitude, the wearable device 100 may obtain the blood pressure value based on the parameter, for example, the first PPG signal or the second PPG signal.

In some embodiments, S1308 and S1309 may not be performed. After prompting the user to increase or decrease the pressing force, the wearable device 100 may directly collect the second PPG signal, and determine whether the amplitude of the second PPG signal is greater than the preset amplitude. When the amplitude of the second PPG signal is less than the preset amplitude, the wearable device 100 continues to prompt, based on the pressure value collected by the pressure sensor in the first button, the user to increase or decrease the pressing force until the amplitude of the second PPG signal is greater than the preset amplitude.

In some embodiments, the third threshold and the fourth threshold may be the same. The third threshold or the fourth threshold may be a pressure value corresponding to pressing force when the amplitude of the PPG signal reaches a maximum amplitude. When determining that the amplitude of the first PPG signal is less than the preset amplitude, the wearable device 100 may obtain the pressure value collected by the pressure sensor in the first button. When the pressure value is less than the third threshold or the fourth threshold, the wearable device 100 may prompt the user to increase the pressing force. When the pressure value is greater than the third threshold or the fourth threshold, the wearable device 100 may prompt the user to decrease the pressing force. After the user adjusts the pressing force, the wearable device 100 continues to obtain the second PPG signal collected by the PPG sensor in the first button. When the amplitude of the second PPG signal is greater than the preset amplitude, the procedure ends. When the amplitude of the second PPG signal is less than the preset amplitude, the wearable device 100 continues to prompt, based on the pressure value collected by the pressure sensor, the user to increase or decrease the pressing force until the amplitude of the second PPG signal is greater than the preset amplitude.

In some embodiments, when determining that the amplitude of the first PPG signal is less than the preset amplitude, the wearable device 100 may not adjust the magnitude of the pressing force based on the pressure value collected by the pressure sensor in the first button.

In a possible implementation, when determining that the amplitude of the first PPG signal is less than the preset amplitude, the wearable device 100 may prompt the user to increase the pressing force. After the user increases the pressing force, if the amplitude of the PPG signal collected by the PPG sensor in the first button keeps decreasing, the wearable device 100 may determine that the amplitude of the PPG signal is less than the preset value because the current pressing force of the user is too large, and the wearable device 100 may further prompt the user to decrease the pressing force until the amplitude of the PPG signal is greater than the preset value, and keep the current pressing force to measure blood pressure. After the user increases the pressing force, if the amplitude of the PPG signal collected by the PPG sensor in the first button gradually increases, the wearable device 100 may prompt the user to continue to increase the pressing force until the amplitude of the PPG signal is greater than the preset value, and keep the current pressing force to measure blood pressure.

In another possible implementation, when determining that the amplitude of the first PPG signal is less than the preset amplitude, the wearable device 100 may prompt the user to decrease the pressing force. After the user decreases the pressing force, if the amplitude of the PPG signal collected by the PPG sensor in the first button keeps decreasing, the wearable device 100 may determine that the amplitude of the PPG signal is less than the preset value because the current pressing force of the user is too small, and the wearable device 100 may further prompt the user to increase the pressing force until the amplitude of the PPG signal is greater than the preset value, and keep the current pressing force to measure blood pressure. After the user decreases the pressing force, if the amplitude of the PPG signal collected by the PPG sensor in the first button gradually increases, the wearable device 100 may prompt the user to continue to decrease the pressing force until the amplitude of the PPG signal is greater than the preset value, and keep the current pressing force to measure blood pressure.

**In some embodiments, the wearable device 100 may first monitor a magnitude of the pressure value corresponding to the pressing force, so that the pressure value is between the first threshold and the second threshold, then monitor the amplitude of the PPG signal, and indicate, based on the amplitude of the PPG signal, the user to continue to increase or decrease the pressing force until the amplitude of the PPG signal is greater than the preset value.**

Because different users have different levels of blood vessel contraction, even if the pressure value is between the first threshold and the second threshold, the amplitude of the PPG signal is still less than the preset value. Therefore, after the magnitude of the pressure is adjusted, the amplitude of the PPG signal further needs to be monitored, so that the amplitude of the PPG signal is greater than the preset amplitude. In other words, in this application, the pressing force may be further adjusted with reference to embodiments in FIG. 11 and FIG. 13A and FIG. 13B, so that the amplitude of the PPG signal is greater than the preset value.

Optionally, the third threshold is less than the fourth threshold, the third threshold is greater than the first threshold, and the fourth threshold is less than the second threshold. In other words, the wearable device 100 may indicate, based on the pressure value collected by the pressure sensor in the first button, the user to adjust the pressure value corresponding to the pressing force to a value between the first threshold and the second threshold. When the amplitude of the PPG signal is still less than the preset amplitude, the wearable device 100 may continue to indicate the user to adjust the pressure value corresponding to the pressure to a value between the third threshold and the fourth threshold, so that the amplitude of the PPG signal is greater than the preset amplitude.

In some embodiments, after the user is indicated to adjust the pressure value corresponding to the pressure to the value between the third threshold and the fourth threshold, and the amplitude of the PPG signal is still less than the preset amplitude, the wearable device 100 may continue to indicate the user to adjust the pressing force. When the pressing force is greater than a fifth threshold, the wearable device 100 may indicate the user to decrease the pressing force. When the pressing force is less than the fifth threshold, the wearable device 100 may indicate the user to increase the pressing force until the amplitude of the PPG signal is greater than the preset amplitude. The fifth threshold is greater than the third threshold and is less than the fourth threshold, and the fifth threshold may be a pressure value corresponding to a pressing force when the amplitude of the PPG signal reaches the maximum amplitude.

**2. The wearable device 100 monitors stability of the PPG signal.**

The quality of the PPG signal is not only related to the amplitude of the PPG signal, but also related to the stability of the PPG signal. The wearable device 100 further needs to monitor the stability of the PPG signal. That the PPG signal is stable may mean that a difference between an amplitude of m consecutive frames of PPG signals and the preset amplitude falls within a first difference, that is, the amplitude of the m consecutive frames of PPG signals fluctuates slightly.

In some embodiments, when the wearable device 100 includes a plurality of PPG sensors, that the PPG signal is stable may mean that PPG signals respectively collected by the plurality of PPG sensors are all stable. For example, a PPG sensor is disposed on the button 101 of the wearable device 100, and a PPG sensor is disposed on the case back 105 of the wearable device 100. That a PPG signal is stable may mean that a PPG signal collected by the PPG sensor disposed on the button 101 is stable, and a PPG signal collected by the PPG sensor disposed on the case back 105 is stable.

When determining that the PPG signal is stable, the wearable device 100 may obtain the blood pressure value of the user through estimation based on a parameter, for example, the collected PPG signal.

When determining that the PPG signal is unstable, the wearable device 100 may further determine a reason why the PPG signal is unstable, and indicate the user to take a related measure to eliminate the reason why the PPG signal is unstable, so that the wearable device 100 can obtain the stable PPG signal.

The reason why the PPG signal is unstable may include but is not limited to any one or more of the following: the user is in a motion state, the user is coughing, speaking, or breathing rapidly, or the like. The reason why the PPG signal is unstable may further include another factor. This is not limited in this application.

In some embodiments, the wearable device 100 may obtain motion data collected by a motion sensor and a sound signal collected by a microphone, and determine, based on the motion data and/or the sound signal collected by the microphone, the reason why the PPG signal is unstable.

For example, when determining, based on the motion data, that the user is currently in the motion state, or determining that the user is in the motion state in a previous period of time, the wearable device 100 may display a user interface shown in FIG. 15A. The user interface shown in FIG. 15A includes prompt information "Do not move, and keep still", and the prompt information indicates that the user needs to keep still during blood pressure measurement, to improve stability of the PPG signal collected by the wearable device 100, and improve accuracy of estimating blood pressure of the user by the wearable device 100 based on the PPG signal.

For example, when determining, based on the sound signal collected by the microphone, that the user is coughing, speaking, or breathing rapidly, or the like, the wearable device 100 may display a user interface shown in FIG. 15B. The user interface shown in FIG. 15B includes prompt information "Do not speak, and keep still", and the prompt information indicates that the user needs to keep still during blood pressure measurement, to improve stability of the PPG signal collected by the wearable device 100, and improve accuracy of estimating blood pressure of the user by the wearable device 100 based on the PPG signal.

For example, when determining, based on the motion data, that the user is not in the motion state, and determining, based on the sound signal collected by the microphone, that the user is not coughing, speaking, or breathing rapidly, or the like, the wearable device 100 may prompt the user to wait for a period of time before measuring blood pressure.

For another example, in the second blood pressure measurement solution, the user wears the wearable device 100, and uses a finger to press the first button, and a pressure signal and first heart rate data (for example, PPG signals) of the user may be collected based on a heart rate sensor, a pressure sensor, and an electrocardiogram sensor on the first button. The PPG signals may include a plurality of PPG signals under multi-wavelength light sources (yellow light, blue light, and the like) and electrocardiogram data (for example, ECG signals). The pressing force of the finger remains in a range (for example, from 40 gF to 70 gF), PPG signals and ECG signals with different wavelengths are continuously collected for a period of time, and a blood pressure value is obtained based on at least one or more features shown below.

It should be noted that the feature may be an independent waveform feature, or may be a joint feature of the PPG signal and the ECG signal. For example, the feature may include: an independent waveform feature, for example, a moment and an amplitude of a QRS complex of the ECG, and waveform features, periods, peaks, and falling edges/rising edges of a plurality of PPG signals; and a joint feature of the PPG and the ECG, for example, obtaining a relationship between moments of different feature points such as a peak-to-peak time interval and a P/T wave peak-to-PPG peak time interval.

For another example, the wearable device 100 may include a third blood pressure measurement method. The third blood pressure measurement method may be implemented by using a third blood pressure measurement component. The third blood pressure measurement component may include a second heart rate sensor, or a second heart rate sensor and the pressure sensor. The pressure sensor is configured to collect a pressure signal in contact with the case back 105.

Optionally, when blood pressure is measured based on the third blood pressure measurement method, the user may maintain a blood pressure measurement posture shown in FIG. 3C, for example, an arm hanging down; or the user may maintain any posture without using a specific posture.

In some embodiments, when blood pressure is measured based on the third blood pressure measurement method, the user may alternatively maintain the blood pressure measurement posture shown in FIG. 3A, or maintain the blood pressure measurement posture similar to that shown in FIG. 3B, but a finger of the user does not need to press or touch the button 101.

Comfort and accuracy of the foregoing three blood pressure measurement methods are different.

For example, for the first blood pressure measurement method, in inflation and deflation processes of the bulb, a user blood vessel is squeezed, and user experience is poor. However, measuring blood pressure by the wearable device 100 based on the first blood pressure measurement method has high accuracy.

For the second blood pressure measurement method, the user needs to actively press the button 101, and comfort of the second blood pressure measurement method is better than that of the first blood pressure measurement method.

For the third blood pressure measurement method, the wearable device 100 may measure blood pressure without awareness of the user, and comfort of the third blood pressure measurement method is better than that of the second blood pressure measurement method.

In conclusion, comfort of the third blood pressure measurement method is better than comfort of the second blood pressure measurement method, and comfort of the second blood pressure measurement method is better than comfort of the first blood pressure measurement method.

In some embodiments, accuracy of the first blood pressure measurement method is higher than accuracy of the second blood pressure measurement method, and accuracy of the second blood pressure measurement method is higher than accuracy of the third blood pressure measurement method.

Based on this feature, the wearable device 100 may obtain first information, and determine a first blood pressure measurement solution based on the first information. The blood pressure measurement solution may include but is not limited to any one or more of the first blood pressure measurement method, the second blood pressure measurement method, and the third blood pressure measurement method.

In addition to the foregoing three blood pressure measurement methods, the wearable device 100 may further include more other blood pressure measurement methods. Based on different conditions, a corresponding blood pressure measurement method is determined to measure blood pressure of the user.

Optionally, the wearable device 100 may also measure blood pressure of the user based on different conditions in combination with a plurality of blood pressure measurement methods. When the blood pressure of the user is measured based on the plurality of blood pressure measurement methods, the user may maintain one posture to measure the blood pressure, or the user may change different postures to measure the blood pressure.

Optionally, after the first blood pressure measurement solution is executed, the wearable device 100 may provide, based on data obtained through measurement and blood pressure results, a comprehensive blood pressure evaluation result of the user that includes but not limited to a maximum value, a minimum value, and an average value of systolic pressure and diastolic pressure during measurement, a blood pressure change trend diagram formed based on the measurement results, a valid data proportion, and other analysis results; and provide proper suggestions for the user, for example, an exercise suggestion or a rest suggestion.

In the method, when the wearable device 100 needs to accurately measure the blood pressure of the user, the wearable device 100 may measure the blood pressure based on a blood pressure measurement method with high accuracy. In another time period, the wearable device 100 may measure the blood pressure based on a blood pressure measurement method with better comfort. In other words, the wearable device 100 may use different blood pressure measurement methods at different time, to monitor the blood pressure of the user. This can improve both accuracy of blood pressure measured by the wearable device 100 and comfort of the user during blood pressure measurement.

In some embodiments, if the wearable device 100 does not generate the first blood pressure measurement solution, the wearable device 100 may alternatively receive a user operation to select any one or more of the first blood pressure measurement method, the second blood pressure measurement method, and the third blood pressure measurement method to measure blood pressure.

In some embodiments, the wearable device 100 may alternatively measure the blood pressure based on a plurality of blood pressure measurement methods.

For example, the wearable device 100 may measure the blood pressure based on the first blood pressure measurement method and the second blood pressure measurement method.

In a possible implementation, the user may maintain the blood pressure measurement posture shown in FIG. 3A, and obtain a 1^{st} blood pressure value through measurement based on the first blood pressure measurement method. Then, the user maintains the blood pressure measurement posture shown in FIG. 3B, and obtains a 2^{nd} blood pressure value based on the second blood pressure measurement method. Alternatively, the user may maintain the blood pressure measurement posture shown in FIG. 3B, and obtain a 1^{st} blood pressure value based on the second blood pressure measurement method. Then, the user maintains the blood pressure measurement posture shown in FIG. 3A, and obtains a 2^{nd} blood pressure value based on the first blood pressure measurement method. The wearable device 100 may obtain a current blood pressure value of the user based on the 1^{st} blood pressure value and the 2^{nd} blood pressure value.

In another possible implementation, the user may maintain a blood pressure measurement posture shown in FIG. 3D, that is, the user presses the button 101 with one finger, and keeps the wrist wearing the wearable device 100 flush with the heart.

In a possible implementation, when the user maintains the blood pressure measurement posture shown in FIG. 3D, the user obtains a 1^{st} blood pressure value through measurement based on the first blood pressure measurement method, and then obtains a 2^{nd} blood pressure value based on the second blood pressure measurement method; or the user obtains a 1^{st} blood pressure value based on the second blood pressure measurement method, and then obtains a 2^{nd} blood pressure value through measurement based on the first blood pressure measurement method. The wearable device 100 may obtain a current blood pressure value of the user based on the 1^{st} blood pressure value and the 2^{nd} blood pressure value.

In another possible implementation, the user may maintain the blood pressure measurement posture shown in FIG. 3D, and obtain two blood pressure values based on the first blood pressure measurement method and the second blood pressure measurement method. The wearable device 100 may obtain a current blood pressure value of the user based on the two blood pressure values. It should be noted that obtaining the two blood pressure values based on the first blood pressure measurement method and the second blood pressure measurement method may mean that a time interval between measurement time of the first blood pressure measurement method and measurement time of the second blood pressure measurement method is less than a first value. For example, the first value may be 5 seconds. The first value may alternatively be another value. This is not limited in this application.

For another example, the wearable device 100 may measure the blood pressure based on the first blood pressure measurement method and the third blood pressure measurement method.

In a possible implementation, the user may maintain the blood pressure measurement posture shown in FIG. 3A, and obtain a 1^{st} blood pressure value through measurement based on the first blood pressure measurement method. Then, the user maintains the blood pressure measurement posture shown in FIG. 3C, and obtains a 2^{nd} blood pressure value based on the third blood pressure measurement method. Alternatively, the user may maintain the blood pressure measurement posture shown in FIG. 3C, and obtain a 1^{st} blood pressure value based on the third blood pressure measurement method. Then, the user maintains the blood pressure measurement posture shown in FIG. 3A, and obtains a 2^{nd} blood pressure value based on the first blood pressure measurement method. The wearable device 100 may obtain a current blood pressure value of the user based on the 1^{st} blood pressure value and the 2^{nd} blood pressure value.

In another possible implementation, the user may maintain the blood pressure measurement posture shown in FIG. 3A, and obtain two blood pressure values based on the first blood pressure measurement method and the third blood pressure measurement method. The wearable device 100 may obtain a current blood pressure value of the user based on the two blood pressure values. It should be noted that obtaining the two blood pressure values based on the first blood pressure measurement method and the third blood pressure measurement method may mean that a time interval between measurement time of the first blood pressure measurement method and measurement time of the third blood pressure measurement method is less than a first value. For example, the first value may be 5 seconds. The first value may alternatively be another value. This is not limited in this application.

For another example, the wearable device 100 may measure the blood pressure based on the second blood pressure measurement method and the third blood pressure measurement method.

In a possible implementation, the user may maintain the blood pressure measurement posture shown in FIG. 3B, and obtain a 1^{st} blood pressure value through measurement based on the second blood pressure measurement method. Then, the user maintains the blood pressure measurement posture shown in FIG. 3C, and obtains a 2^{nd} blood pressure value based on the third blood pressure measurement method. Alternatively, the user may maintain the blood pressure measurement posture shown in FIG. 3C, and obtain a 1^{st} blood pressure value based on the third blood pressure measurement method. Then, the user maintains the blood pressure measurement posture shown in FIG. 3B, and obtains a 2^{nd} blood pressure value based on the second blood pressure measurement method. The wearable device 100 may obtain a current blood pressure value of the user based on the 1^{st} blood pressure value and the 2^{nd} blood pressure value.

In another possible implementation, the user may maintain the blood pressure measurement posture shown in FIG. 3B, and obtain two blood pressure values based on the second blood pressure measurement method and the third blood pressure measurement method. The wearable device 100 may obtain a current blood pressure value of the user based on the two blood pressure values. It should be noted that obtaining the two blood pressure values based on the second blood pressure measurement method and the third blood pressure measurement method may mean that a time interval between measurement time of the second blood pressure measurement method and measurement time of the third blood pressure measurement method is less than a first value. For example, the first value may be 5 seconds. The first value may alternatively be another value. This is not limited in this application. In other words, when blood pressure is measured based on the second blood pressure measurement method and the third blood pressure measurement method, the user may maintain one posture, or may maintain different postures.

For another example, the wearable device 100 may measure the blood pressure based on the first blood pressure measurement method, the second blood pressure measurement method, and the third blood pressure measurement method.

In a possible implementation, the user may separately maintain the blood pressure measurement posture shown in FIG. 3A, the blood pressure measurement posture shown in FIG. 3B, and the blood pressure measurement posture shown in FIG. 3C, obtain a 1^{st} blood pressure value through measurement based on the first blood pressure measurement method, obtain a 2^{nd} blood pressure value through measurement based on the second blood pressure measurement method, and obtain a 3^{rd} blood pressure value based on the third blood pressure measurement method, to obtain a current blood pressure value of the user. A measurement sequence of the first blood pressure measurement method, the second blood pressure measurement method, and the third blood pressure measurement method is not limited.

In another possible implementation, the user may maintain the blood pressure measurement posture shown in FIG. 3D, and obtain three blood pressure values based on the first blood pressure measurement method, the second blood pressure measurement method, and the third blood pressure measurement method. The wearable device 100 may obtain a current blood pressure value of the user based on the three blood pressure values. It should be noted that obtaining the three blood pressure values based on the first blood pressure measurement method, the second blood pressure measurement method, and the third blood pressure measurement method may mean that a time interval between measurement time of any two of the first blood pressure measurement method, the second blood pressure measurement method, and the third blood pressure measurement method is less than a first value. For example, the first value may be 5 seconds. The first value may alternatively be another value. This is not limited in this application.

Optionally, this application is described by using an example in which first values are the same. The first values may alternatively be different. For example, time intervals of the measurement time of any two blood pressure measurement methods may be different. For example, a time interval between measurement time of the first blood pressure measurement method and measurement time of the second blood pressure measurement method is less than the first value, a time interval between the measurement time of the second blood pressure measurement method and measurement time of the third blood pressure measurement method is less than a second value, and the first value is different from the second value.

In addition to the foregoing plurality of blood pressure measurement methods, the wearable device 100 may further include more other blood pressure measurement methods. This is not limited in this application.

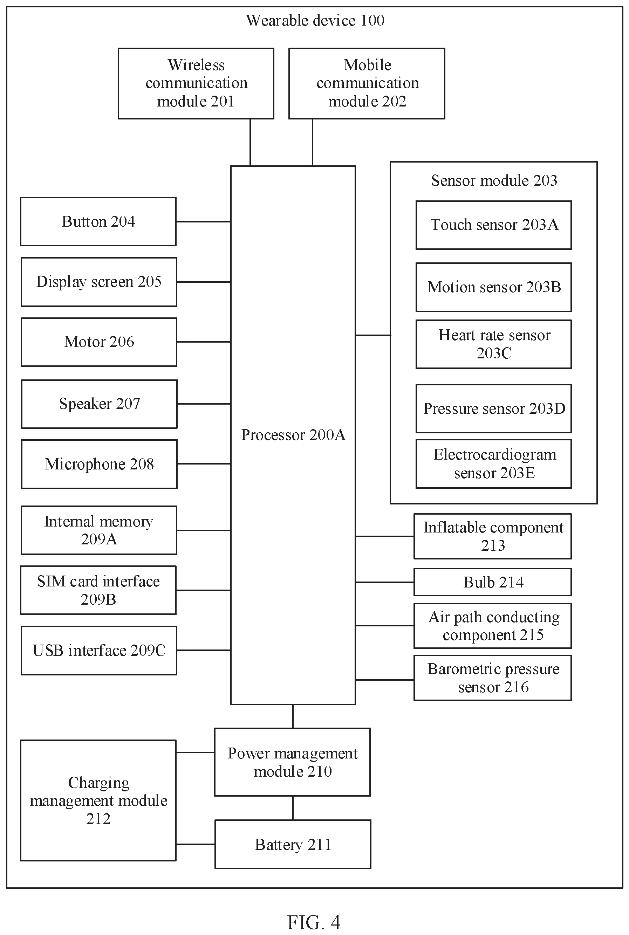

FIG. 4 is a diagram of a hardware structure of a wearable device 100.

The wearable device 100 may be a band, a watch, or another device. A specific type of the wearable device is not particularly limited in embodiments of this application. In embodiments of this application, only an example in which the wearable device 100 is a watch is used for description.

As shown in FIG. 4, the wearable device 100 may include: a processor 200A, a wireless communication module 201, a mobile communication module 202, a sensor module 203, a button 204, a display screen 205, a motor 206, a speaker 207, a microphone 208, an internal memory 209A, a SIM card interface 209B, a USB interface 209C, a power management module 210, a battery 211, and a charging management module 212. The sensor module 203 may include a touch sensor 203A, a motion sensor 203B, a heart rate sensor 203C, a pressure sensor 203D, and an electrocardiogram sensor 203E.

The processor 200A may include one or more processing units. For example, the processor 200A may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, and/or a neural network processing unit (neural-network processing unit, NPU). Different processing units may be independent components, or may be integrated into one or more processors.

The wireless communication module 201 may provide a solution, applied to the wearable device, to wireless communication including a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, and the like. The wireless communication module 201 may be one or more components integrating at least one communication processing module. The wireless communication module 201 receives an electromagnetic wave through the antenna, performs frequency modulation and filtering on an electromagnetic wave signal, and sends a processed signal to the processor 200A. The wireless communication module 201 may further receive a to-be-sent signal from the processor 200A, perform frequency modulation and amplification on the signal, and convert a processed signal into an electromagnetic wave for radiation through the antenna.

The mobile communication module 202 may provide a solution applied to the wearable device for wireless communication such as 2G/3G/4G/5G. The mobile communication module 202 may include at least one filter, a switch, a power amplifier, a low noise amplifier (low noise amplifier, LNA), and the like. The mobile communication module 202 may receive an electromagnetic wave through the antenna, perform processing such as filtering or amplification on the received electromagnetic wave, and transmit a processed electromagnetic wave to the modem processor for demodulation. In some embodiments, at least some functional modules of the mobile communication module 202 may be disposed in the processor 200A. In some embodiments, at least some functional modules of the mobile communication module 202 may be disposed in a same component as at least some modules of the processor 200A.

A wireless communication function of the wearable device may be implemented by the mobile communication module 202, the wireless communication module 201, the modem processor, the baseband processor, and the like.

The touch sensor 203A is also referred to as a "touch panel". The touch sensor 203A may be disposed on the display screen of the wearable device 100, and the touch sensor 203A and the display screen form a touch screen, which is also referred to as a "touchscreen". The touch sensor 203A is configured to monitor a touch operation performed on or near the touch sensor. The touch sensor may transfer the detected touch operation to the processor, to determine a touch event type. A visual output related to the touch operation may be provided on the display screen.

The motion sensor 203B may obtain motion data, and determine, based on the motion data, a non-static state of the user that wears the wearable device. The non-static state may include a static state, a non-static state, and the like. The motion sensor may include but is not limited to a gyroscope sensor, an acceleration sensor, and the like. An angular velocity of the wearable device 100 around three axes (namely, x, y, and z axes) may be determined by using the gyroscope sensor. The acceleration sensor may detect magnitudes of accelerations of the wearable device 100 in various directions (usually, on three axes). When the wearable device 100 is still, a magnitude and a direction of gravity may be detected.

The heart rate sensor 203C is configured to collect heart rate data, and the heart rate data may be used to estimate a blood pressure value of the user. The heart rate sensor 203C may be any one of a PPG sensor, a laser sensor, an ultrasonic sensor, and a magnetic induction sensor. There may be one or more heart rate sensors 203C on the wearable device 100. Optionally, the heart rate sensor 203C may include a first heart rate sensor and a second heart rate sensor.

The pressure sensor 203D is configured to sense a pressure signal, and may convert the pressure signal into an electrical signal. There are a plurality of types of pressure sensors 203D such as a resistive pressure sensor, an inductive pressure sensor, and a capacitive pressure sensor. The capacitive pressure sensor may include at least two parallel plates made of conductive materials. When force is applied to the pressure sensor 203D, capacitance between electrodes changes, and the wearable device 100 determines pressure intensity based on the capacitance change. There may be one or more pressure sensors 203D on the wearable device 100.

The electrocardiogram sensor 203E is configured to collect electrocardiogram information. The electrocardiogram information may be used with the heart rate data to estimate the blood pressure value of the user. The electrocardiogram sensor 203E may be an ECG sensor. There may be one or more electrocardiogram sensors 203E on the wearable device 100.

In some embodiments, the electrocardiogram sensor 203E may include a plurality of electrodes, for example, a first electrode and a second electrode. The heart rate sensor 203C and the pressure sensor 203D may be disposed on the button 101, the first electrode may be disposed on a surface of the button 101, and the second electrode may be disposed on a surface of the case back 105. When a pressing operation is performed on the button 101, the first electrode and the second electrode form a loop. The wearable device 100 may collect an ECG signal via the first electrode and the second electrode, collect a PPG signal of a finger by using the heart rate sensor 203C on the button 101, and collect a pressure signal of the finger by using the pressure sensor 203D on the button 101. Then, the wearable device 100 may estimate the blood pressure value of the user based on the pressure signal of the finger, the PPG signal of the finger, and the ECG signal. Optionally, the wearable device 100 may alternatively detect intensity of pressing the button 101 by the user, and indicate the user to adjust a magnitude of pressing force, so that the wearable device 100 can collect PPG data with good quality, to improve accuracy of an estimated blood pressure value.

Optionally, the wearable device 100 may not include the electrocardiogram sensor 203E, and estimates the blood pressure value of the user based on the pressure signal of the finger and the PPG signal of the finger.

Optionally, the wearable device 100 may not include the electrocardiogram sensor 203E and the pressure sensor 203D, and estimates the blood pressure value of the user based on the PPG signal of the finger.

In some embodiments, the electrocardiogram sensor 203E may include a plurality of electrodes, for example, a first electrode and a second electrode. The heart rate sensor 203C and the pressure sensor 203D may be disposed on the case back 105. The first electrode may be disposed on a surface of the button 101, or the first electrode may be disposed on another position of the watch body, for example, a surface of the side surface of the wearable device 100 or a surface of the display surface 104. When a pressing operation is performed on the button 101 or the another position of the watch body, the first electrode and the second electrode form a loop. The wearable device 100 may collect an ECG signal via the first electrode and the second electrode, collect a PPG signal of the wrist by using the heart rate sensor 203C on the case back 105, and collect a pressure signal of the wrist by using the pressure sensor 203D on the case back 105. Then, the wearable device 100 may estimate the blood pressure value of the user based on the pressure signal of the wrist, the PPG signal of the wrist, and the ECG signal. Optionally, the wearable device 100 may alternatively detect a magnitude of the pressure signal of the wrist, and indicate the user to adjust tightness of the wearable component 103, so that the wearable device 100 can collect PPG data with good quality, to improve accuracy of an estimated blood pressure value.

Optionally, the wearable device 100 may not include the electrocardiogram sensor 203E, and estimates the blood pressure value of the user based on the pressure signal of the wrist and the PPG signal of the wrist.

The button 204 includes a power button and the like. Optionally, the wearable device 100 may not include the electrocardiogram sensor 203E and the pressure sensor 203D, and estimates the blood pressure value of the user based on the PPG signal of the wrist.

The button 204 may be a mechanical button or may be a touch button. The wearable device may receive an input on the button, and generate a button signal input related to user setting and function control of the wearable device. Optionally, the button 204 may include the button 101 and the button 102 shown in FIG. 1. Optionally, the button 204 may include only the button 101 or the button 102 shown in FIG. 1.

The display screen 205 is configured to display an image, a video, or the like. The display screen 205 includes a display panel. The display panel may be a liquid crystal display (liquid crystal display, LCD), an organic light-emitting diode (organic light-emitting diode, OLED), an active-matrix organic light emitting diode or an active-matrix organic light-emitting diode (active-matrix organic light-emitting diode, AMOLED), a flexible light-emitting diode (flexible light-emitting diode, FLED), a quantum dot light-emitting diode (quantum dot light-emitting diode, QLED), or the like. In some embodiments, the wearable device may include one or N display screens 205, where N is a positive integer greater than 1.

The motor 206 may generate a vibration prompt. The motor 206 may be configured to produce an incoming call vibration prompt or a touch vibration feedback. For example, touch operations performed on different applications (for example, photographing and audio playback) may correspond to different vibration feedback effect. The motor 206 may also correspond to different vibration feedback effect for touch operations performed on different areas of the display screen 205. In some embodiments, the wearable device may alternatively be configured to prompt or interact with the user based on different vibration feedbacks of the motor due to magnitudes of pressure of the pressure sensor 203D.

The speaker 207, also referred to as a "loudspeaker", is configured to convert an audio electrical signal into a sound signal. The wearable device may listen to music or answer a call in a hands-free mode by using the speaker 207.

The microphone 208, also referred to as a "mike" or a "mic", is configured to convert a sound signal into an electrical signal. When making a call or sending voice information, the user may make a sound near the microphone 208 through the mouth of the user, to input a sound signal to the microphone 208. At least one microphone 208 may be disposed in the wearable device.

The internal memory 209A may be configured to store computer-executable program code. The executable program code includes instructions. The processor 200A runs the instructions stored in the internal memory 209A, to perform various function applications and data processing of the wearable device. V may include a program storage area and a data storage area. The program storage area may store an operating system, an application required by at least one function (for example, a voice playing function or an image playing function), and the like. The data storage area may store data (for example, audio data) created when the internal memory 209A is used, and the like. In addition, the internal memory 209A may include a high-speed random access memory, or may include a non-volatile memory, for example, at least one disk storage device, a flash memory, or a universal flash storage (universal flash storage, UFS).

The SIM card interface 209B is configured to connect to a SIM card. The SIM card may be inserted into the SIM card interface 209B or removed from the SIM card interface 209B, to implement contact with and separation from the wearable device. The wearable device may support one or N SIM card interfaces, where N is a positive integer greater than 1. The SIM card interface 209B may support a nano SIM card, a micro SIM card, a SIM card, and the like. A plurality of cards may be inserted into the same SIM card interface 209B at the same time. The plurality of cards may be of a same type or different types. The SIM card interface 209B may also be compatible with different types of SIM cards. The SIM card interface 209B may also be compatible with an external memory card. The wearable device interacts with a network through the SIM card, to implement functions such as calling and data communication. In some embodiments, an eSIM, namely, an embedded SIM card, is used for the wearable device. The eSIM card may be embedded into the wearable device, and cannot be separated from the wearable device. In some embodiments, the wearable device may not include the SIM card interface 209B.

The USB interface 209C is an interface that conforms to a USB standard specification, and may be specifically a mini USB interface, a micro USB interface, a USB type-C interface, or the like. The USB interface 209C may be configured to connect to a charger to charge the wearable device, or may be configured to transmit data between the USB interface 209C and a peripheral device, or may be configured to connect to a headset for playing audio through the headset. The interface may be further configured to connect to another electronic device such as an AR device.

The power management module 210 is configured to connect to the battery 211, the charging management module 212, and the processor 200A. The power management module 210 receives an input from the battery 211 and/or the charging management module 212, and supplies power to the processor 200A, the internal memory 209A, the display screen 205, the wireless communication module 201, and the like. The power management module 210 may be further configured to monitor parameters such as a battery capacity, a quantity of battery cycles, and a battery health status (electric leakage or impedance). In some other embodiments, the power management module 210 may alternatively be disposed in the processor 200A. In some other embodiments, the power management module 210 and the charging management module 212 may alternatively be disposed in a same component.

The charging management module 212 is configured to receive a charging input from a charger. The charger may be a wireless charger or a wired charger. In some embodiments of wired charging, the charging management module 212 may receive a charging input from the wired charger through the USB interface 209C. In some embodiments of wireless charging, the charging management module 212 may receive a wireless charging input through a wireless charging coil of the wearable device. When charging the battery 211, the charging management module 212 may further supply power to the wearable device through the power management module 210.

The inflatable component 213 is used for inflation and deflation. In some embodiments of this application, the wearable device 100 inflates the bulb 214 by using the inflatable component 213. The inflatable component 213 and the bulb 214 may be connected through an air path conducting component 215. After the bulb 214 is inflated, the bulb 214 is configured to squeeze a blood vessel of the user, so that the barometric pressure sensor 216 may obtain pressure data, to determine a blood pressure value of the user.

It should be noted that the air path conducting component 215 may be an independent component, or the air path conducting component 215 may be an air path formed by combining other hardware modules, or the air path conducting component 215 may be a part of another component, for example, a part of the inflatable component 213, or a part of the bulb 214.

The barometric pressure sensor 216 is configured to obtain pressure data. In some embodiments of this application, the wearable device 100 may measure atmospheric pressure in the bulb 214 by using the barometric pressure sensor 216, and obtain pressure data. In some embodiments of this application, some components of the barometric pressure sensor 216 are located inside the bulb 214, and are configured to sense barometric pressure in the bulb 214.

The bulb 214 is attached to a side that is of the wearable component 103 and that is close to the body. The inflatable component 213 is connected to the bulb 214 through the air path conducting component 215. The bulb 214 may be attached to only one side of the wearable component 103, and the bulb 214 may be located above an artery of the wrist of the user, for example, above the radial artery.

Optionally, the inflatable component 213 may be located inside the watch body of the wearable device 100, the bulb 214 may be connected to a buckle of the bracelet, and the bulb 214 is connected to the watch face through a vent cap. Correspondingly, the bulb 214 may be separated from the bracelet, or may be separated from the watch face.

In some embodiments, the wearable device 100 may further include a magnetic sensor. For example, the magnetic sensor may be a Hall sensor. In some embodiments of this application, the wearable device 100 may determine, by using the magnetic sensor, whether the bulb 214 on the wearable device 100 is removed. For example, a magnet may be configured on the bulb 214 or the wearable component 103 connected to the bulb 214. The wearable device 100 may determine, by using the magnetic sensor, magnetic flux generated by the magnet on the bulb 214 or the wearable component 103, to determine whether the bulb 214 on the wearable device 100 is removed.

It can be understood that the structure shown in this embodiment of the present invention does not constitute a specific limitation on the wearable device. In some other embodiments of this application, the wearable device may include more or fewer components than those shown in the figure, or some components may be combined, or some components may be split, or the components may be arranged differently. The components shown in the figure may be implemented by hardware, software, or a combination of software and hardware.

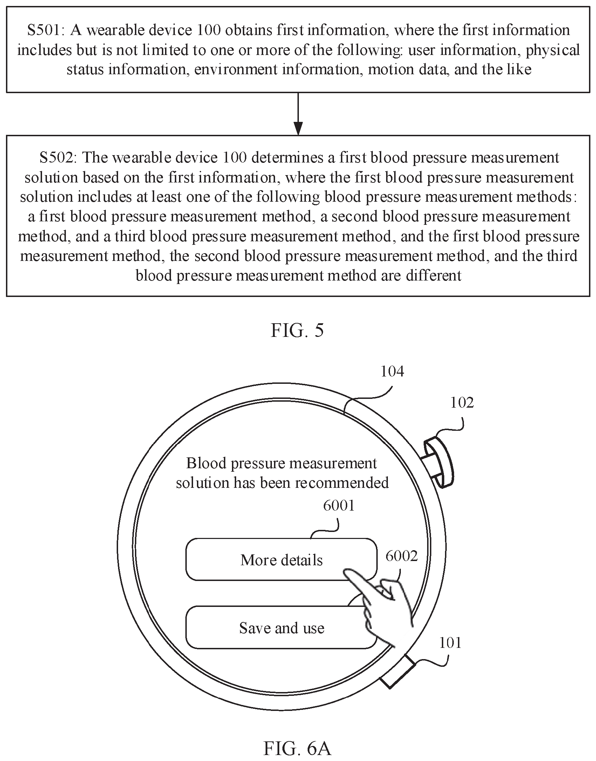

FIG. 5 is a schematic flowchart of a method in which a wearable device 100 determines a blood pressure measurement solution.

S501: The wearable device 100 obtains first information, where the first information includes but is not limited to one or more of the following: user information, physical status information, environment information, motion data, and the like.

The first information includes one or more of the following: the user information, the environment information, the motion data, and the like.

The user information includes one or more of the following: blood pressure, a heart rate, a body temperature, a pulse, a respiratory rate, blood glucose, a smoking history, a drinking history, a history of disease, a medication history, lifestyle habits, gender, age, a height, a weight, and the like.

The environment information includes any one or more of the following: climate, a temperature, altitude, season, weather, time, date, and the like.

A manner in which the wearable device 100 obtains the first information includes but is not limited to any one or more of the following:

**Manner 1:** A user actively inputs the first information to the wearable device 100.

In a possible implementation, when the user needs the wearable device 100 to recommend a blood pressure measurement solution to the user, the user may actively input the first information to the wearable device 100, so that the wearable device 100 can recommend the blood pressure measurement solution to the user based on the first information.

In another possible implementation, the first information may alternatively be input by the user to the wearable device 100 irregularly.

**Manner 2:** The wearable device 100 obtains the first information locally or from another electronic device.

The first information may be stored in the wearable device 100, and the wearable device 100 may obtain the first information locally.

The wearable device 100 may establish a communication connection to another electronic device (for example, a mobile phone), and the wearable device 100 may obtain the first information from the electronic device. The first information stored in the electronic device may be actively input by the user.

Optionally, the blood pressure in the user information may be obtained by the wearable device 100 through real-time measurement, or may be a historical blood pressure value. The historical blood pressure value may be obtained based on a plurality of blood pressure values measured by the user in a past period of time, for example, an average value of the plurality of blood pressure values, or a maximum value of blood pressure values measured by the user in a past period of time, or a minimum value of blood pressure values measured by the user in a past period of time.

Optionally, the blood pressure in the user information may alternatively be obtained based on one or more of a first blood pressure measurement method, a second blood pressure measurement method, and a third blood pressure measurement method.

The wearable device 100 may alternatively obtain the first information in another manner. This is not limited in this application.

S502: The wearable device 100 determines a first blood pressure measurement solution based on the first information, where the first blood pressure measurement solution includes at least one of the following blood pressure measurement methods: the first blood pressure measurement method, the second blood pressure measurement method, and the third blood pressure measurement method, and the first blood pressure measurement method, the second blood pressure measurement method, and the third blood pressure measurement method are different.

After obtaining the first information, the wearable device 100 may determine the first blood pressure measurement solution based on the first information. The first blood pressure measurement solution includes at least one of the following blood pressure measurement methods: the first blood pressure measurement method, the second blood pressure measurement method, and the third blood pressure measurement method, and the first blood pressure measurement method, the second blood pressure measurement method, and the third blood pressure measurement method are different.

The first blood pressure measurement solution may further include information such as a quantity of times of the first blood pressure measurement method, a quantity of times of the second blood pressure measurement method, a quantity of times of the third blood pressure measurement method, measurement time of the first blood pressure measurement method, measurement time of the second blood pressure measurement method, measurement time of the third blood pressure measurement method, and execution time of the first blood pressure measurement solution. The execution time of the first blood pressure measurement solution may be at least 1 day, for example, 1 day, 7 days, or 10 days.

Optionally, the measurement time of the blood pressure measurement method may include but is not limited to start time of a single measurement, end time of a single measurement, duration of a single measurement, and a time interval between two adjacent blood pressure measurements.

The quantity of times of the first blood pressure measurement method, the quantity of times of the second blood pressure measurement method, and the quantity of times of the third blood pressure measurement method are all different, or partially the same, or all the same.

The measurement time of the first blood pressure measurement method, the measurement time of the second blood pressure measurement method, and the measurement time of the third blood pressure measurement method are all different, or partially the same, or all the same.

The wearable device 100 obtains different first blood pressure measurement solutions based on different first information. For example, first information of different users is different, and the wearable device 100 may generate different blood pressure measurement solutions to different users. For a same user, different first blood pressure measurement solutions may be obtained based on different first information.

For example, for a user whose blood pressure is normal, in the blood pressure measurement solution, the quantity of times of the first blood pressure measurement method may be less than the quantity of times of the second blood pressure measurement method, and the quantity of times of the second blood pressure measurement method may be less than the quantity of times of the third blood pressure measurement method.

For another example, a quantity of times of a first blood pressure measurement method in a blood pressure measurement solution for a user with normal blood pressure may be less than a quantity of times of a first blood pressure measurement method in a blood pressure measurement solution for a user with abnormal blood pressure. Therefore, a quantity of times of the first blood pressure measurement method may be appropriately increased for a user with abnormal blood pressure, to accurately monitor blood pressure of the user with abnormal blood pressure.

For example, the first blood pressure measurement solution may include: At 7:00 a.m., blood pressure is measured based on the first blood pressure measurement method. Between 7:00 a.m. and 9:00 a.m., blood pressure is measured based on the third blood pressure measurement method. At 9:00 a.m., blood pressure is measured based on the second blood pressure measurement method. At 9:30 a.m., blood pressure is measured based on the second blood pressure measurement method. At 10:00 a.m., blood pressure is measured based on the second blood pressure measurement method. Between 9:30 a.m. and 10:00 a.m., blood pressure is measured based on the third blood pressure measurement method. Between 10:00 a.m. and 6:00 p.m., blood pressure is measured based on the third blood pressure measurement method. At 6:00 p.m., blood pressure is measured based on the first blood pressure measurement method. Between 6:00 p.m. and 7:00 p.m., blood pressure is measured based on the third blood pressure measurement method. At 7:00 p.m., blood pressure is measured based on the second blood pressure measurement method. At 10:00 p.m., blood pressure is measured based on the second blood pressure measurement method. Between 10:00 p.m. and 7:00 a.m. the next day, blood pressure is measured based on the third blood pressure measurement method. After the user goes to sleep at night, it is inconvenient for the user to use the first blood pressure measurement method or the second blood pressure measurement method. In this case, the wearable device 100 may measure blood pressure of the user based on the third blood pressure measurement method, to avoid disturbing the user's rest. Optionally, if the user is in a sleep state, and the user is not easily affected by pressure applied by a bulb, the wearable device 100 may alternatively measure blood pressure of the user based on the first blood pressure measurement method when the user is in the sleep state at night.

In some embodiments, after obtaining the first blood pressure measurement solution, the wearable device 100 may prompt the user to view, modify, and save the first blood pressure measurement solution.

An occasion at which the wearable device 100 recommends the first blood pressure measurement solution includes but is not limited to any one or more of the following:
**Manner 1:** After the user inputs the first information and the first blood pressure measurement solution is generated based on the first information, the wearable device 100 may recommend the first blood pressure measurement solution to the user.

In a possible implementation, when the user needs the wearable device 100 to recommend a blood pressure measurement solution to the user, the user may actively input the first information to the wearable device 100. After obtaining the first information and obtaining the first blood pressure measurement solution based on the first information, the wearable device 100 may prompt the user to view the first blood pressure measurement solution.

**Manner 2:** The wearable device 100 periodically/occasionally recommends the first blood pressure measurement solution to the user.

The first information may continuously change, and the wearable device 100 may periodically/occasionally generate the first blood pressure measurement solution based on the first information. After obtaining the first blood pressure measurement solution, the wearable device 100 may prompt the user to view the first blood pressure measurement solution.

The wearable device 100 may further recommend the first blood pressure measurement solution to the user in another manner. This is not limited in this application.

Optionally, the wearable device 100 may alternatively prompt, at time when the user does not use the wearable device 100, the user to view the first blood pressure measurement solution, to avoid affecting normal usage of the wearable device 100 by the user.

Optionally, the wearable device 100 may alternatively prompt, when the user is in a non-sleep state, the user to view the first blood pressure measurement solution, to avoid affecting the user's rest. For example, the wearable device 100 may determine, based on collected information such as a heart rate and motion data, whether the user is in a sleep state.

FIG. 6A to FIG. 6C are diagrams in which a wearable device 100 prompts a user to view, modify, and save a first blood pressure measurement solution.

After obtaining the first blood pressure measurement solution, the wearable device 100 may display a user interface shown in FIG. 6A. The user interface shown in FIG. 6A includes an option 6001 and an option 6002, and the user may view content of the first blood pressure measurement solution via the option 6001. The user may also save and use the first blood pressure measurement solution via the option 6002.

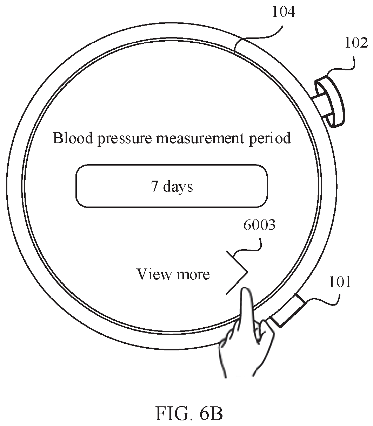

For example, as shown in FIG. 6A, the wearable device 100 may receive an input operation of the user for the option 6001, for example, a single tap. In response to the input operation of the user, the wearable device 100 may display a user interface shown in FIG. 6B.

For example, as shown in FIG. 6B, the user interface shown in FIG. 6B shows function duration of the first blood pressure measurement solution. For example, the function duration may be 7 days. The user interface shown in FIG. 6B further includes an option 6003, and the user may continue to view other content of the first blood pressure measurement solution via the option 6003.

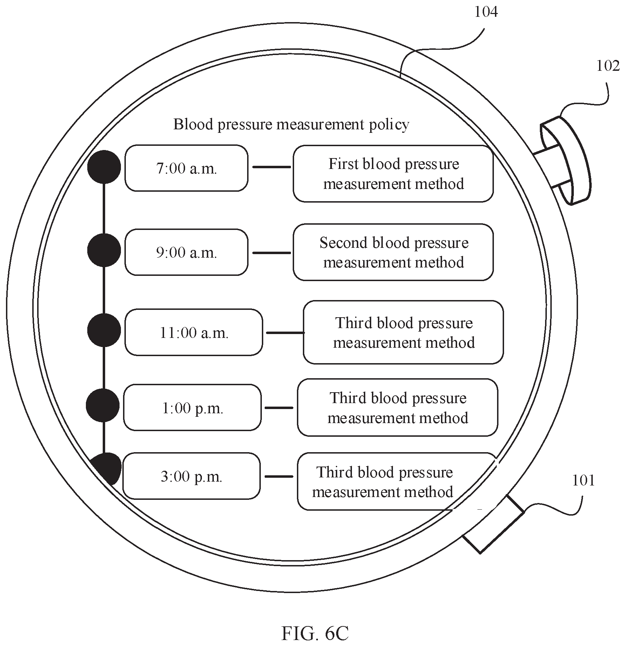

For example, as shown in FIG. 6B, the wearable device 100 may receive an input operation of the user for the option 6003, for example, a single tap. In response to the input operation of the user, the wearable device 100 may display a user interface shown in FIG. 6C.

For example, as shown in FIG. 6C, the user interface shown in FIG. 6C includes a blood pressure measurement policy of the first blood pressure measurement solution, and the blood pressure measurement policy may include blood pressure measurement methods at different time. For example, the blood pressure measurement policy shown in FIG. 6C may include: At 7:00 a.m., blood pressure is measured based on the first blood pressure measurement method. At 9:00 a.m., blood pressure is measured based on the second blood pressure measurement method. At 11:00 a.m., blood pressure is measured based on the third blood pressure measurement method. At 1:00 p.m., blood pressure is measured based on the third blood pressure measurement method. At 3:00 p.m., blood pressure is measured based on the third blood pressure measurement method. Alternatively, the user may scroll the user interface shown in FIG. 6C to view another blood pressure measurement policy of the first blood pressure measurement solution.

In some embodiments, the wearable device 100 may also receive a user operation to change the blood pressure measurement policy of the first blood pressure measurement solution, for example, change time of the blood pressure measurement method, or change the blood pressure measurement method. The wearable device 100 may also receive a user operation to save the modified first blood pressure measurement solution. The wearable device 100 may also receive a user operation to save the unmodified first blood pressure measurement solution.

In some embodiments, the user may also modify content of the first blood pressure measurement method. For example, the wearable device 100 may receive an input operation, for example, a single tap, of the user on the option of the second blood pressure measurement method corresponding to 9:00 a.m. shown in FIG. 6C. In response to the input operation of the user, the wearable device 100 may further receive an operation of the user to modify the "second blood pressure measurement method" corresponding to 9:00 a.m. into the "third blood pressure measurement method". Then, the wearable device 100 may display a user interface similar to that shown in FIG. 6C, but 9:00 a.m. corresponds to the "third blood pressure measurement method".

In some embodiments, after obtaining the first blood pressure measurement solution, the wearable device 100 may obtain a blood pressure value of the user, and determine, based on the blood pressure value of the user, whether the first blood pressure measurement solution needs to be modified. When the blood pressure of the user is abnormal, the wearable device 100 may change the first blood pressure measurement solution, so that the modified first blood pressure measurement solution can more accurately monitor the blood pressure of the user. When the blood pressure of the user is normal, the wearable device 100 may appropriately reduce a quantity of measurement times of the first blood pressure measurement method or the second blood pressure measurement method, so that the modified first blood pressure measurement solution improves comfort of the user.

Optionally, before using the first blood pressure measurement solution, the wearable device 100 may determine whether the first blood pressure measurement solution needs to be modified. The wearable device 100 may alternatively determine, in a process of executing the first blood pressure measurement solution, whether the first blood pressure measurement solution needs to be modified.

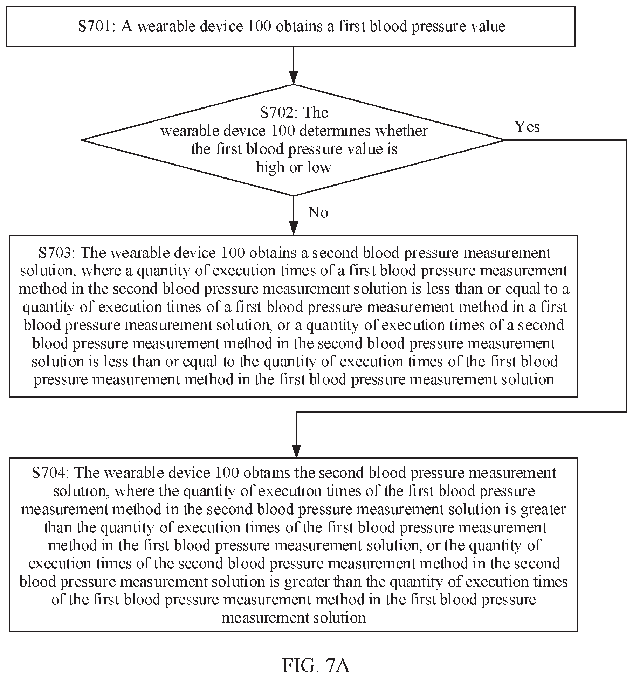

FIG. 7A is a schematic flowchart of a method in which a wearable device 10 determines, based on a blood pressure value of a user, whether to modify a first blood pressure measurement solution.

S701: The wearable device 100 obtains a first blood pressure value.

In a possible implementation, the first blood pressure value may be manually input by the user to the wearable device 100. For example, the user obtains the first blood pressure value through measurement by using a blood pressure gauge, and the user may manually input the first blood pressure value to the wearable device 100.

In another possible implementation, the first blood pressure value may be obtained by the wearable device 100 based on a blood pressure measurement method with high accuracy, for example, obtained based on a first blood pressure measurement method. A blood pressure measurement result of the first blood pressure measurement method is closer to an actual blood pressure value of the user. For example, after obtaining the first blood pressure measurement solution, or before starting to measure blood pressure based on the first blood pressure measurement solution, the wearable device 100 may obtain the first blood pressure value based on the first blood pressure measurement method, to determine a blood pressure status of the user, and determine, based on the first blood pressure value, whether to adjust the first blood pressure measurement solution.

The wearable device 100 may further obtain the first blood pressure value in another manner. This is not limited in this application.

S702: The wearable device 100 determines whether the first blood pressure value is high or low.

When it is determined that the first blood pressure value is high or low, it indicates that the blood pressure of the user is abnormal, and the wearable device 100 may change the first blood pressure measurement solution.

When it is determined that the first blood pressure value is not high and is not low, that is, the blood pressure of the user is normal, the wearable device 100 may also change the first blood pressure measurement solution, so that comfort of the user is improved by using the modified first blood pressure measurement solution. Alternatively, the wearable device 100 may not change the first blood pressure measurement solution.

The first blood pressure value being high may mean that the first blood pressure value is greater than a first threshold. For example, the first threshold may be 120 millimeters of mercury (mmHg). The first blood pressure value being low may mean that the first blood pressure value is less than a second threshold. For example, the second threshold may be 60 mmHg. The first blood pressure value being normal may mean that the first blood pressure value is greater than the second threshold and less than the first threshold. For example, the first blood pressure value is greater than 60 mmHg and less than 120 mmHg.

Optionally, the first threshold and the second threshold may be statistical data, or may be average values of blood pressure of the user of the wearable device 100 in a period of time.

When the first blood pressure value is normal, S703 is performed.

When the first blood pressure value is high or low, S704 is performed.

S703: The wearable device 100 obtains a second blood pressure measurement solution, where a quantity of execution times of a first blood pressure measurement method in the second blood pressure measurement solution is less than or equal to a quantity of execution times of the first blood pressure measurement method in the first blood pressure measurement solution, or a quantity of execution times of a second blood pressure measurement method in the second blood pressure measurement solution is less than or equal to a quantity of execution times of a second blood pressure measurement method in the first blood pressure measurement solution.

In some embodiments, when it is determined that the first blood pressure value is normal, the wearable device 100 may also store the first blood pressure measurement solution, that is, the first blood pressure measurement solution does not need to be modified.

In some embodiments, when it is determined that the first blood pressure value is normal, the wearable device 100 may appropriately reduce a quantity of execution times of a blood pressure measurement method that affects comfort of the user, for example, reduce the quantity of execution times of the first blood pressure measurement method or the quantity of execution times of the second blood pressure measurement method, to obtain the second blood pressure measurement solution.

In some embodiments, when determining that the first blood pressure value is normal, the wearable device 100 may alternatively replace a blood pressure measurement method that affects comfort of the user with a blood pressure measurement method that provides better comfort for the user. For example, the first blood pressure measurement method is replaced with the second blood pressure measurement method or a third blood pressure measurement solution, or the second blood pressure measurement method is replaced with the third blood pressure measurement solution.

Optionally, the wearable device 100 may obtain the second blood pressure measurement solution based on the first blood pressure value.

Optionally, the wearable device 100 may alternatively modify the first blood pressure measurement solution based on the first blood pressure value, to obtain the second blood pressure measurement solution.

In a possible implementation, when it is determined that the first blood pressure value is normal, that the wearable device 100 reduces the quantity of execution times of the first blood pressure measurement method may include but is not limited to any one of the following cases:
Case 1: When the quantity of execution times of the first blood pressure measurement method in the second blood pressure measurement solution is less than or equal to the quantity of execution times of the first blood pressure measurement method in the first blood pressure measurement solution, the quantity of execution times of the second blood pressure measurement method in the second blood pressure measurement solution is greater than the quantity of execution times of the second blood pressure measurement method in the first blood pressure measurement solution, and a quantity of execution times of a third blood pressure measurement method in the second blood pressure measurement solution is the same as a quantity of execution times of a third blood pressure measurement method in the first blood pressure measurement solution.

Case 2: When the quantity of execution times of the first blood pressure measurement method in the second blood pressure measurement solution is less than or equal to the quantity of execution times of the first blood pressure measurement method in the first blood pressure measurement solution, the quantity of execution times of the second blood pressure measurement method in the second blood pressure measurement solution is the same as the quantity of execution times of the second blood pressure measurement method in the first blood pressure measurement solution, and the quantity of execution times of the third blood pressure measurement method in the second blood pressure measurement solution is greater than the quantity of execution times of the third blood pressure measurement method in the first blood pressure measurement solution.

In another possible implementation, that the wearable device 100 reduces the quantity of execution times of the second blood pressure measurement method when determining that the first blood pressure value is normal may include: When the quantity of execution times of the second blood pressure measurement method in the second blood pressure measurement solution is less than or equal to the quantity of execution times of the first blood pressure measurement method in the second blood pressure measurement solution, the quantity of execution times of the first blood pressure measurement method in the second blood pressure measurement solution is less than the quantity of execution times of the first blood pressure measurement method in the first blood pressure measurement solution, and the quantity of execution times of the third blood pressure measurement method in the second blood pressure measurement solution is greater than the quantity of execution times of the third blood pressure measurement method in the first blood pressure measurement solution.

In another embodiment, when determining that the first blood pressure value is normal, the wearable device 100 may alternatively remain the quantity of execution times of the first blood pressure measurement method unchanged, increase the quantity of execution times of the second blood pressure measurement method, and reduce the quantity of execution times of the third blood pressure measurement method.

S704: The wearable device 100 obtains the second blood pressure measurement solution, where the quantity of execution times of the first blood pressure measurement method in the second blood pressure measurement solution is greater than the quantity of execution times of the first blood pressure measurement method in the first blood pressure measurement solution, or the quantity of execution times of the second blood pressure measurement method in the second blood pressure measurement solution is greater than the quantity of execution times of the second blood pressure measurement method in the first blood pressure measurement solution.

When determining that the first blood pressure value is abnormal, the wearable device 100 may increase the quantity of execution times of the first blood pressure measurement method, or increase the quantity of execution times of the second blood pressure measurement method.

Optionally, when the quantity of execution times of the first blood pressure measurement method in the second blood pressure measurement solution is greater than the quantity of execution times of the first blood pressure measurement method in the first blood pressure measurement solution, or the quantity of execution times of the second blood pressure measurement method in the second blood pressure measurement solution is greater than the quantity of execution times of the second blood pressure measurement method in the first blood pressure measurement solution, the quantity of execution times of the third blood pressure measurement method in the second blood pressure measurement solution is the same as the quantity of execution times of the third blood pressure measurement method in the first blood pressure measurement solution; or the quantity of execution times of the third blood pressure measurement method in the second blood pressure measurement solution is less than the quantity of execution times of the third blood pressure measurement method in the first blood pressure measurement solution.

Optionally, after the wearable device 100 obtains the first blood pressure measurement solution, and the wearable device 100 executes the first blood pressure measurement solution for first duration, the wearable device 100 may obtain a plurality of blood pressure values. The wearable device 100 may update the first blood pressure measurement solution based on the plurality of blood pressure values, to obtain the third blood pressure measurement solution.

For example, when determining, based on the plurality of blood pressure values, that the blood pressure of the user is normal, the wearable device 100 may appropriately reduce the quantity of measurement times of the first blood pressure measurement method in the first blood pressure measurement solution or the quantity of measurement times of the second blood pressure measurement method, to improve comfort of blood pressure measurement by the wearable device 100.

For another example, when determining, based on the plurality of blood pressure values, that the blood pressure of the user is abnormal, the wearable device 100 may appropriately increase the quantity of measurement times of the first blood pressure measurement method in the first blood pressure measurement solution or increase the quantity of measurement times of the second blood pressure measurement method. This improves accuracy of blood pressure measured by the wearable device 100, to more accurately monitor the blood pressure of the user with abnormal blood pressure.

Optionally, after the wearable device 100 updates the blood pressure measurement solution, for example, after obtaining the second blood pressure measurement solution or the third blood pressure measurement solution, the wearable device 100 may prompt the user to view the updated blood pressure measurement solution.

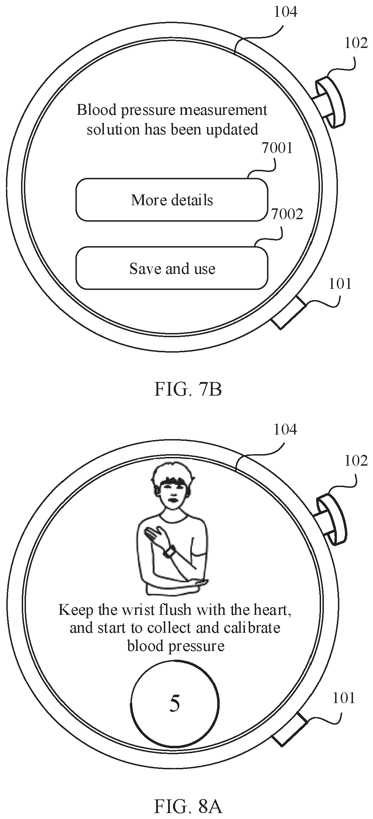

For example, the wearable device 100 may display a user interface shown in FIG. 7B. The user interface shown in FIG. 7B includes prompt information "Blood pressure measurement solution has been updated". The user interface shown in FIG. 7B further includes an option 7001 and an option 7002, and the user may view content of the updated blood pressure measurement solution via the option 7001. The user may also save and use the updated blood pressure measurement solution via the option 7002.

In some embodiments, after obtaining the first blood pressure measurement solution, the wearable device 100 may also verify accuracy of the second blood pressure measurement method or the third blood pressure measurement method. When accuracy of the second blood pressure measurement method or the third blood pressure measurement method is not high, the wearable device 100 may reduce the quantity of execution times of the second blood pressure measurement method or the quantity of execution times of the third blood pressure measurement method. For example, the wearable device 100 may collect physiological data based on the second blood pressure measurement method or the third blood pressure measurement method. When the physiological data collected based on the second blood pressure measurement method or the third blood pressure measurement method differs greatly from historical physiological data, it indicates that accuracy of the second blood pressure measurement method or the third blood pressure measurement method is not high. In this case, the wearable device 100 may reduce the quantity of execution times of the second blood pressure measurement method or the quantity of execution times of the third blood pressure measurement method.

When the physiological data collected based on the second blood pressure measurement method or the third blood pressure measurement method does not greatly differ from the historical physiological data, it indicates that accuracy of the second blood pressure measurement method or the third blood pressure measurement method meets a requirement. In this case, the wearable device 100 may remain the quantity of execution times of the second blood pressure measurement method unchanged or increase the quantity of execution times of the second blood pressure measurement method, or remain the quantity of execution times of the third blood pressure measurement method unchanged or increase the quantity of execution times of the third blood pressure measurement method.

After determining the blood pressure measurement solution, the wearable device 100 may measure blood pressure of the user based on the first blood pressure measurement method, the second blood pressure measurement method, and the third blood pressure measurement method respectively, to monitor the blood pressure of the user.

The second blood pressure measurement method and the third blood pressure measurement method estimate a blood pressure value of the user based on the collected physiological data. To improve accuracy of blood pressure measured based on the second blood pressure measurement method and the third blood pressure measurement method, the wearable device 100 may calibrate a model used to estimate blood pressure in the second blood pressure measurement method and the third blood pressure measurement method.

In some embodiments, in addition to blood pressure measurement based on one blood pressure measurement method, the wearable device 100 may also obtain a plurality of blood pressure values based on a plurality of blood pressure measurement methods, and use an average value of the plurality of blood pressure values as a currently detected blood pressure value.

In a model calibration process and a blood pressure measurement process, the wearable device 100 may prompt the user to maintain a posture to complete the model calibration process or the blood pressure measurement process. Therefore, the user can complete the model calibration process or the blood pressure measurement process without changing the posture, which reduces user operations. In some embodiments, the model calibration process or the blood pressure measurement process may also be referred to as an awareness scenario.

The wearable device 100 may also prompt the user to separately complete the model calibration process or the blood pressure measurement process in different postures. In this way, the user may change different postures to complete the model calibration process or the blood pressure measurement process, and the user maintains each posture for short time. In some embodiments, the model calibration process or the blood pressure measurement process may also be referred to as an unawareness scenario.

The following describes calibration processes and blood pressure measurement processes of a plurality of blood pressure measurement methods based on an awareness scenario and an unawareness scenario.

### 1. Awareness scenario

**The awareness scenario may include an awareness calibration scenario and an awareness blood pressure measurement scenario.**

### 1. Awareness calibration scenario

### (1) Calibrate a second target model of the second blood pressure measurement method

### A. Initial calibration of the second target model

The second target model may be a model used to estimate a blood pressure value in the second blood pressure measurement method. An input of the second target model is second information, and an output of the second target model is a blood pressure value. The second information may be collected by the second blood pressure measurement component. The second information may include first heart rate data, or first heart rate data and electrocardiogram data, or first heart rate data, pressure data, and electrocardiogram data.

Optionally, in addition to the second information, the input of the second target model may further include other information such as gyroscope and acceleration data. This is not limited in this application.

Before the second target model is calibrated for the first time, if the user has not used the wearable device 100 to measure blood pressure, and the wearable device 100 does not store historical blood pressure information, or the user has used the wearable device 100 to measure blood pressure, but a small amount of historical blood pressure information is stored in the wearable device 100, the wearable device 100 may collect the second information for a plurality of times, and determine, based on the second information collected for the plurality of times, whether the user is in a static state. When determining that the user is in the static state, the wearable device 100 calibrates the second target model again. When determining that the user is in a non-static state, the wearable device 100 may also prompt the user to calibrate the second target model when the user is in the static state later. Therefore, the wearable device 100 may calibrate the second target model based on the data collected when the user is in the static state, to improve accuracy of calibrating the second target model.

For example, a plurality of times may be two times. This is not limited to collecting the second information for two times. Alternatively, more second information may be collected. This is not limited in this application.

The following describes an occasion at which the wearable device 100 calibrates the second target model for the first time.

**Occasion 1:** When detecting a specific event, the wearable device 100 prompts the user to calibrate the second target model.

For example, the specific event may be an event of detecting that the wearable device 100 is powered on. When the second target model has not been calibrated, after detecting that the wearable device 100 is powered on, the wearable device 100 may prompt the user to calibrate the second target model.

For example, the specific event may alternatively be an event of detecting that the wearable device 100 is to measure blood pressure based on the second target model. For example, blood pressure measurement time is time t1, and in a period of time before the time t1, for example, time t2, the time t2 is earlier than the time t1. The wearable device 100 may prompt the user to calibrate the second target model.

This is not limited to the foregoing specific event, and the specific event may be another event. This is not limited in this application.

**Occasion 2:** The wearable device 100 receives a user operation to calibrate the second target model.

In some embodiments, the user may also actively trigger the wearable device 100 to calibrate the second target model.

**Occasion 3:** The wearable device 100 receives an instruction for starting to calibrate the second target model from another electronic device, and starts to calibrate the second target model.

In some embodiments, the wearable device 100 may establish a communication connection relationship with the another electronic device (for example, a mobile phone or a tablet computer). When detecting a user operation used to calibrate the second target model, the another electronic device may send, to the wearable device 100, the instruction for starting to calibrate the second target model. When receiving the instruction for starting to calibrate the second target model, the wearable device 100 may start to calibrate the second target model.

In addition to the foregoing several occasions at which the second target model starts to be calibrated, the wearable device 100 may alternatively start to calibrate the second target model at another occasion. This is not limited in this application either.

For example, when starting to calibrate the second target model, the wearable device 100 may first prompt the user to maintain a posture shown in FIG. 8A, and collect third information based on the first blood pressure measurement method. The third information includes a blood pressure value A (including at least a systolic blood pressure SBP and a diastolic blood pressure DBP) and/or pressure data A, and the pressure data A may be used to obtain the blood pressure value A.

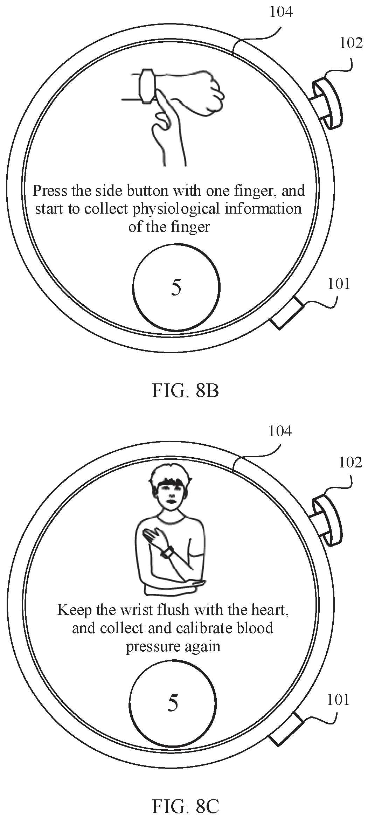

After obtaining the third information, the wearable device 100 may further prompt the user to maintain a posture shown in FIG. 8B, and collect second information based on the second blood pressure measurement method.

After obtaining the second information, the wearable device 100 may further prompt the user to maintain a posture shown in FIG. 8C, and collect fourth information based on the first blood pressure measurement method. The fourth information includes a blood pressure value B and/or pressure data B, and the pressure data B may be used to obtain the blood pressure value B.

After obtaining the third information and the fourth information, the wearable device 100 may determine whether a difference between the third information and the fourth information is less than a threshold. When the difference is less than the threshold, it indicates that the user is in the static state, and the wearable device 100 may calibrate the second target model based on the second information, the third information, and/or the fourth information. When the difference is greater than the threshold, it indicates that the user is in the non-static state, and the wearable device 100 prompts the user to calibrate the second target model later.

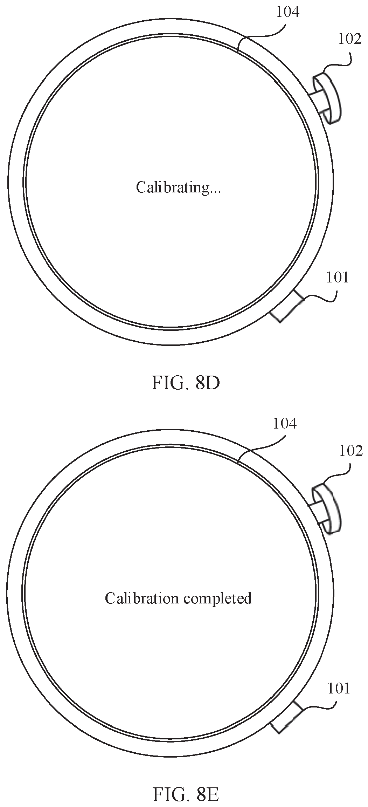

Optionally, when the wearable device 100 calibrates the second target model, the wearable device 100 may display prompt information shown in FIG. 8D. The prompt information includes "Calibrating...", and the prompt information prompts the user that the wearable device 100 is calibrating the second target model.

Optionally, after calibration of the second target model is completed, the wearable device 100 may also display prompt information shown in FIG. 8E, and the prompt information includes "Calibration completed". The prompt information prompts the user that calibration of the second target model has been completed.

It should be noted that, in another possible implementation, the wearable device 100 may first separately display the postures shown in FIG. 8A and FIG. 8C, to collect the third information and the fourth information. After determining that the difference between the third information and the fourth information is less than the threshold, the wearable device 100 then displays the posture shown in FIG. 8B, to prompt the user to collect the second information, and calibrate the second target model based on the second information, the third information, and/or the fourth information.

In another possible implementation, the wearable device 100 may display the posture shown in FIG. 8B, to collect the second information, and then display the postures shown in FIG. 8A and FIG. 8C, to collect the third information and the fourth information. After determining that the difference between the third information and the fourth information is less than the threshold, the wearable device 100 then calibrates the second target model based on the second information, the third information, and/or the fourth information.

When the third information includes the blood pressure value A, and the fourth information includes the blood pressure value B, that the difference between the third information and the fourth information is less than the threshold may mean that a difference between the blood pressure value A and the blood pressure value B is less than the threshold. When the third information includes the pressure data A, and the fourth information includes the pressure data B, that the difference between the third information and the fourth information is less than a threshold may mean that a difference between the pressure data A and the pressure data B is less than the threshold. For example, a difference between an evaluation parameter extracted based on the pressure data A and an evaluation parameter extracted based on the pressure data B may be less than the threshold.

That the wearable device 100 calibrates the second target model based on the second information, the third information, and/or the fourth information may include: The wearable device 100 inputs the second information to the second target model, and the second target model outputs a blood pressure value C. Then, the wearable device 100 obtains a blood pressure value D. The blood pressure value D may be the blood pressure value A, or the blood pressure value B, or an average value of the blood pressure value A and the blood pressure value B. After obtaining the blood pressure value C and the blood pressure value D, the wearable device 100 may calibrate the second target model based on a difference between the blood pressure value C and the blood pressure value D. The blood pressure value output by the calibrated second target model based on the second information is more accurate than the blood pressure value output, based on the second information, by the second target model that is not calibrated.

In some embodiments, when determining, based on the motion data collected by the motion sensor, whether the user is in the static state or the non-static state, the wearable device 100 may also collect the third information or the fourth information based on the first blood pressure measurement method for one time. A sequence of obtaining the third information or the fourth information and the second information is not limited.

In some embodiments, when the difference between the third information and the fourth information is greater than the second threshold, the wearable device 100 may also calibrate the second target model based on the collected data. The wearable device 100 may obtain the blood pressure value D based on the pressure data in the third information and/or the fourth information, to improve accuracy of the blood pressure value D obtained by the wearable device 100 when the user is in the non-static state.

The wearable device 100 may obtain the blood pressure value D based on the pressure data in the third information and/or the fourth information in any one of the following manners. This is not limited.

Manner 1: When a condition A is met, the blood pressure value D is obtained by the wearable device 100 based on the second information and the pressure data A in the third information, or the blood pressure value D may be obtained by the wearable device 100 based on the second information and the pressure data B in the fourth information, or the blood pressure value D may be obtained by the wearable device 100 based on the second information, the pressure data A in the third information, and the pressure data B in the fourth information.

Manner 2: When a condition B is met, the blood pressure value D is obtained by the wearable device 100 based on the pressure data A in the third information, the second blood pressure value may be obtained by the wearable device 100 based on the pressure data B in the fourth information, or the second blood pressure value may be obtained by the wearable device 100 based on the pressure data A in the third information and the pressure data B in the fourth information.

For example, the condition A may be that the user is in the non-static state and quality of the pressure data A or the pressure data B does not meet a requirement, and the condition B may be that the user is in the non-static state and quality of the pressure data A and the pressure data B meets the requirement.

It should be noted that the condition A and the condition B may alternatively be other conditions. This is not limited in this application.

In some embodiments, the wearable device 100 may alternatively determine the blood pressure value D in another manner. For example, when a condition C is met, the blood pressure value D is obtained by the wearable device 100 based on the second information, the pressure data A in the third information, and/or the pressure data B in the fourth information. The condition C may be that the user is in the non-static state. It should be noted that the condition C may alternatively be another condition. This is not limited in this application.

After obtaining the blood pressure value C and the blood pressure value D, the wearable device 100 calibrates the second target model based on a deviation between the blood pressure value C and the blood pressure value D.

Based on the method, the wearable device 100 may prompt the user to change different postures to obtain a plurality of pieces of information, to calibrate the second target model, and the user maintains each posture for short time.

### B. Non-initial calibration of the second target model

When a large amount of historical blood pressure information is stored in the wearable device 100, the wearable device 100 may collect third information based on the first blood pressure measurement method, and determine, based on the third information and the historical blood pressure information, whether the user is in a static state. If a difference between the third information and the historical blood pressure information is small, it indicates that the user is in the static state, and the wearable device 100 may calibrate the second target model. If the difference between the third information and the historical blood pressure information is large, it indicates that the user is in a non-static state, and the wearable device 100 may alternatively prompt the user to wait for a period of time before calibrating the second target model. In this way, the wearable device 100 only needs to collect the third information for one time, to simplify a calibration procedure.

In some embodiments, when a large amount of historical blood pressure information is stored in the wearable device 100, the wearable device 100 may alternatively collect data for a plurality of times based on the first blood pressure measurement method, and determine, based on the data collected for the plurality of times and the first blood pressure measurement method, whether the user is in the static state.

An occasion at which the wearable device 100 performs non-initial calibration on the second target model may be similar to the occasion at which the wearable device 100 initially calibrates the second target model. Details are not described herein in this application.

In some embodiments, when the wearable device 100 performs non-initial calibration on the second target model, the wearable device 100 may prompt the user to select a calibration mode. The calibration mode may include a standard calibration mode and a simplified calibration mode. The standard calibration mode may mean that the wearable device 100 collects data for a plurality of times based on the first blood pressure measurement method, and determines, based on the data collected for the plurality of times and the first blood pressure measurement method, whether the user is in the static state. The simplified calibration mode may mean that the wearable device 100 collects data, for example, the third information, for one time based on the first blood pressure measurement method, and determines, based on the third information and the historical blood pressure information, whether the user is in the static state.

In this embodiment of this application, an example in which the wearable device 100 collects the third information for one time based on the first blood pressure measurement method in non-initial calibration of the second target model is used for description.

In a possible implementation, the wearable device 100 first prompts the user to maintain the posture shown in FIG. 8A, and collects the third information based on the first blood pressure measurement method. When determining, based on the third information and the historical blood pressure information, that the user is in the static state, the wearable device 100 then prompts the user to maintain the posture shown in FIG. 8B, collects the second information based on the second blood pressure measurement method, and calibrates the second target model based on the third information and the second information.

In another possible implementation, the wearable device 100 first prompts the user to maintain the posture shown in FIG. 8B, and collects the second information based on the second blood pressure measurement method; and then, prompts the user to maintain the posture shown in FIG. 8A, and collects the third information based on the first blood pressure measurement method. When determining, based on the third information and the historical blood pressure information, that the user is in the static state, the wearable device 100 calibrates the second target model based on the third information and the second information.

That the wearable device 100 determines, based on the third information and the historical blood pressure information, whether the user is in the static state may include:

When the third information includes a blood pressure value, for example, the blood pressure value A, and the historical blood pressure information includes a historical blood pressure value, the wearable device 100 needs to determine whether a difference between the blood pressure value A in the third information and the historical blood pressure value is less than a threshold A. When the difference is less than the threshold A, it indicates that the user is currently in the static state. When the difference is greater than the third threshold, it indicates that the user is currently in the non-static state.

When the second information includes pressure data, for example, the pressure data A, and the historical blood pressure information includes historical pressure data, the wearable device 100 needs to determine whether the pressure data A in the third information and the historical pressure data is less than a threshold B. For example, a difference between an evaluation parameter extracted based on the pressure data A and an evaluation parameter extracted based on the pressure data B may be less than the threshold. When the difference is less than the threshold B, it indicates that the user is currently in the static state. When the difference is greater than the threshold B, it indicates that the user is currently in the non-static state.

The threshold A and the threshold B may be the same or may be different.

A principle of calibrating the second target model by the wearable device 100 based on the second information and the third information is similar to a principle of initially calibrating the second target model by the wearable device 100. Details are not described herein in this application.

In some embodiments, when determining, based on the third information and the historical blood pressure information, that the user is in the non-static state, the wearable device 100 may alternatively calibrate the second target model based on the collected data. A specific implementation is similar to a principle in which the wearable device 100 initially calibrates the second target model when the user is in the non-static state. Details are not described herein in this application either.

### (2) Calibrate a first target model of the third blood pressure measurement method

### A. Initial calibration of the first target model

The first target model may be a model used to estimate a blood pressure value in the third blood pressure measurement method. An input of the first target model is fifth information, and an output of the first target model is a blood pressure value. The fifth information may be collected by the third blood pressure measurement component. The fifth information may include but is not limited to second heart rate data.

An implementation of initial calibration of the first target model is similar to that of initial calibration of the second target model, and a difference between initial calibration of the first target model and initial calibration of the second target model lies in that postures of the user are different.

In some embodiments, the wearable device 100 may calibrate the first target model based on a blood pressure value and/or pressure data collected based on the first blood pressure measurement method.

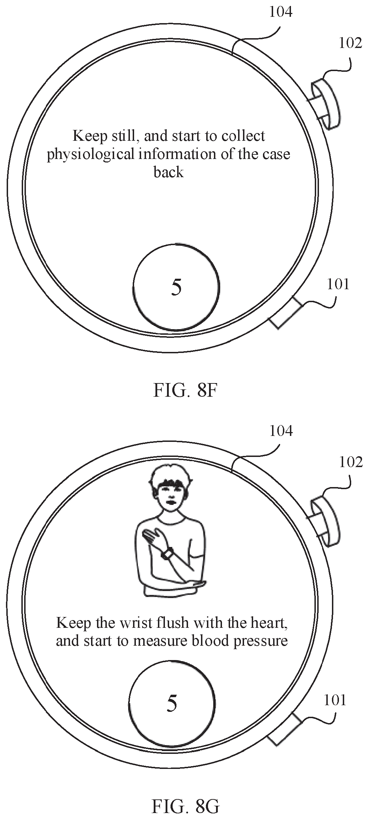

In a possible implementation, the wearable device 100 first prompts the user to maintain the posture shown in FIG. 8A, to obtain the third information. After obtaining the third information, the wearable device 100 then prompts the user to maintain a posture shown in FIG. 8F, to obtain the fifth information. After obtaining the fifth information, the wearable device 100 then prompts the user to maintain the posture shown in FIG. 8C, to obtain the fourth information.

In another possible implementation, the wearable device 100 first prompts the user to maintain the posture shown in FIG. 8F, to obtain the fifth information. After obtaining the fifth information, the wearable device 100 then prompts the user to maintain the posture shown in FIG. 8A, to obtain the third information. After obtaining the third information, the wearable device 100 then prompts the user to maintain the posture shown in FIG. 8C, to obtain the fourth information.

In another possible implementation, the wearable device 100 first prompts the user to maintain the posture shown in FIG. 8A, to obtain the third information. After obtaining the third information, the wearable device 100 then prompts the user to maintain the posture shown in FIG. 8C, to obtain the fourth information. After obtaining the fourth information, the wearable device 100 then prompts the user to maintain the posture shown in FIG. 8F, to obtain the fifth information.

After obtaining the third information, the fourth information, and the fifth information, the wearable device 100 may calibrate the first target model based on the third information, the fourth information, and the fifth information. A specific implementation in which the wearable device 100 calibrates the first target model based on the fifth information, the third information, and/or the fourth information is similar to a specific implementation in which the wearable device 100 initially calibrates the second target model. Details are not described herein in this application.

In some embodiments, when determining, based on the motion data collected by the motion sensor, whether the user is in a static state or a non-static state, the wearable device 100 may also collect the third information or the fourth information based on the first blood pressure measurement method for one time. A sequence of obtaining the third information or the fourth information and the fifth information is not limited.

In another embodiment, the wearable device 100 may alternatively calibrate the first target model based on a blood pressure value collected based on the second blood pressure measurement method.

In a possible implementation, the wearable device 100 first prompts the user to maintain the posture shown in FIG. 8B, to obtain sixth information. The sixth information may include but is not limited to any one or more of the following: heart rate data, electrocardiogram data, pressure data, and a blood pressure value. After obtaining the sixth information for the first time, the wearable device 100 prompts the user to maintain the posture shown in FIG. 8F, to obtain the fifth information. After obtaining the fifth information, the wearable device 100 then prompts the user to maintain the posture shown in FIG. 8B, to obtain seventh information. The seventh information may include but is not limited to any one or more of the following: heart rate data, electrocardiogram data, pressure data, and a blood pressure value.

In another possible implementation, the wearable device 100 first prompts the user to maintain the posture shown in FIG. 8F, to obtain the fifth information. After obtaining the fifth information, the wearable device 100 then prompts the user to maintain the posture shown in FIG. 8B, to obtain the sixth information. After obtaining the sixth information, the wearable device 100 then prompts the user to maintain the posture shown in FIG. 8B, to obtain the seventh information.

In another possible implementation, the wearable device 100 first prompts the user to maintain the posture shown in FIG. 8B, to obtain the sixth information. After obtaining the sixth information, the wearable device 100 then prompts the user to maintain the posture shown in FIG. 8B, to obtain the seventh information. After obtaining the seventh information, the wearable device 100 then prompts the user to maintain the posture shown in FIG. 8F, to obtain the fifth information.

When a difference between the sixth information and the seventh information is less than a threshold, it indicates that the user is in a static state, and the wearable device 100 may calibrate the first target model. When the difference between the sixth information and the seventh information is greater than the threshold, it indicates that the user is in the non-static state, and the wearable device 100 may prompt the user to calibrate the first target model later when the user is in the static state.

A principle of calibrating the first target model by the wearable device 100 based on the sixth information, the seventh information, and the fifth information is similar to a principle of initially calibrating the second target model by the wearable device 100. Details are not described herein in this application.

In some embodiments, when determining, based on the motion data collected by the motion sensor, whether the user is in the static state or the non-static state, the wearable device 100 may also collect the sixth information or the seventh information based on the second blood pressure measurement method for one time. A sequence of obtaining the sixth information or the seventh information and the fifth information is not limited.

### B. Non-initial calibration of the first target model

An implementation of non-initial calibration of the first target model is similar to that of initial calibration of the first target model, and a difference between non-initial calibration of the first target model and non-initial calibration of the first target model lies in that postures of the user are different.

In some embodiments, the wearable device 100 may calibrate the first target model based on a blood pressure value and/or pressure data collected based on the first blood pressure measurement method.

In a possible implementation, the wearable device 100 first prompts the user to maintain the posture shown in FIG. 8A, and collects the third information based on the first blood pressure measurement method. When determining, based on the third information and the historical blood pressure information, that the user is in the static state, the wearable device 100 then prompts the user to maintain the posture shown in FIG. 8F, collects the fifth information based on the third blood pressure measurement method, and calibrates the first target model based on the third information and the fifth information.

In another possible implementation, the wearable device 100 first prompts the user to maintain the posture shown in FIG. 8F, and collects the fifth information based on the third blood pressure measurement method; and then prompts the user to maintain the posture shown in FIG. 8A, and collects the third information based on the first blood pressure measurement method. When determining, based on the third information and the historical blood pressure information, that the user is in the static state, the wearable device 100 calibrates the first target model based on the third information and the fifth information.

A principle of calibrating the first target model by the wearable device 100 based on the third information and the fifth information is similar to a principle of initially calibrating the second target model by the wearable device 100. Details are not described herein in this application. Details are not described herein in this application.

In some embodiments, when determining, based on the third information and the historical blood pressure information, that the user is in the non-static state, the wearable device 100 may alternatively calibrate the second target model based on the collected data. A specific implementation is similar to a principle in which the wearable device 100 initially calibrates the first target model when the user is in the non-static state. Details are not described herein in this application either.

In another embodiment, the wearable device 100 may alternatively calibrate the first target model based on a blood pressure value collected based on the second blood pressure measurement method.

In a possible implementation, the wearable device 100 first prompts the user to maintain the posture shown in FIG. 8B, to obtain the sixth information. When determining, based on the sixth information and the historical blood pressure value, that the user is in the static state, the wearable device 100 then prompts the user to maintain the posture shown in FIG. 8F, to obtain the fifth information, and calibrates the first target model based on the sixth information and the fifth information.

In another possible implementation, the wearable device 100 first prompts the user to maintain the posture shown in FIG. 8F, to obtain the fifth information. After obtaining the fifth information, the wearable device 100 then prompts the user to maintain the posture shown in FIG. 8B, to obtain the sixth information. When determining, based on the sixth information and the historical blood pressure value, that the user is in the static state, the wearable device 100 calibrates the first target model based on the sixth information and the fifth information.

A principle of calibrating the first target model by the wearable device 100 based on the sixth information and the fifth information is similar to a principle of initially calibrating the second target model by the wearable device 100. Details are not described herein in this application.

### 2. Awareness blood pressure measurement scenario

### (1) Measure blood pressure based on the first blood pressure measurement method

When preset time such as first time arrives, the wearable device 100 may prompt the user to start to measure blood pressure based on the first blood pressure measurement method.

For example, the wearable device 100 may display prompt information shown in FIG. 8G. The prompt information may include "Keep the wrist flush with the heart, and start to measure blood pressure". The prompt information is used to prompt the user to measure blood pressure based on the first blood pressure measurement method in a correct posture, to improve accuracy of blood pressure measured based on the first blood pressure measurement method.

In some embodiments, before the wearable device 100 obtains the blood pressure value based on the first blood pressure measurement method, the wearable device 100 may obtain a pulse wave signal at the constant atmospheric pressure and physiological data at the constant atmospheric pressure, determine a blood pressure risk based on the pulse wave signal at the constant atmospheric pressure and the physiological data at the constant atmospheric pressure, and then determine the blood pressure value based on the blood pressure risk and the pressure data. This can improve accuracy of blood pressure measured based on the first blood pressure measurement method.

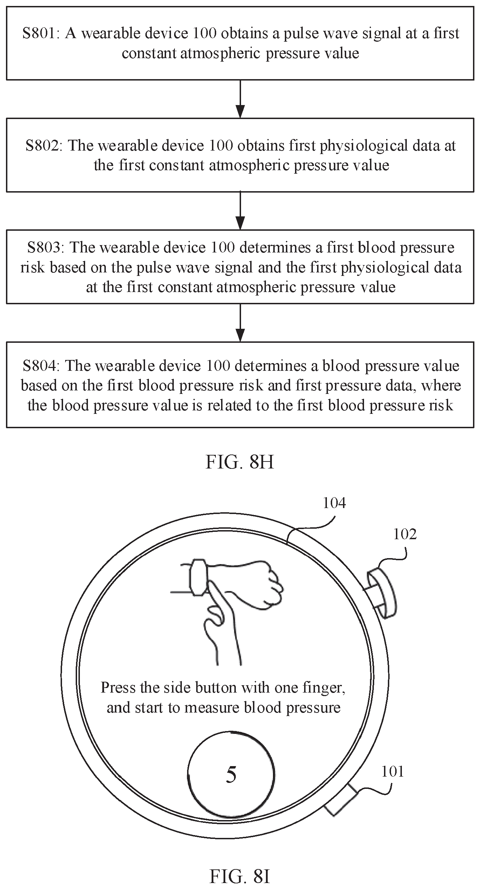

FIG. 8H is a schematic flowchart of a method in which a wearable device 100 obtains a blood pressure value based on a first blood pressure measurement method.

S801: The wearable device 100 obtains a pulse wave signal at a first constant atmospheric pressure value.

When measuring blood pressure based on the first blood pressure measurement method, the wearable device 100 may measure the blood pressure based on an inflation method or a deflation method.

For the inflation method, the wearable device 100 needs to inflate the bulb. When atmospheric pressure in the bulb linearly increases, pressure data is obtained by using a barometric pressure sensor. The pressure data may include bulb pressure and a pulse wave signal corresponding to the bulb pressure. The wearable device 100 may obtain a blood pressure value based on the pressure data.

For the deflation method, the wearable device 100 needs to inflate the bulb; and after atmospheric pressure in the bulb reaches a specific value, starts to deflate the bulb. When the atmospheric pressure in the bulb linearly decreases, the wearable device 100 may obtain pressure data by using the barometric pressure sensor, and obtain a blood pressure value based on the pressure data.

When the atmospheric pressure in the bulb linearly increases or the atmospheric pressure in the bulb linearly decreases, the wearable device 100 may keep the atmospheric pressure in the bulb stable at the first constant atmospheric pressure value. The first constant atmospheric pressure value may be preset. When the atmospheric pressure in the bulb is fixed at the first constant atmospheric pressure value, the wearable device 100 may obtain the pulse wave signal at the first constant atmospheric pressure value by using the atmospheric pressure sensor.

Optionally, a plurality of constant atmospheric pressure values may be set, and pulse wave signals corresponding to the plurality of constant atmospheric pressure values are respectively set.

S802: The wearable device 100 obtains first physiological data at the first constant atmospheric pressure value.

When the atmospheric pressure in the bulb is stable at the first constant atmospheric pressure value, the wearable device 100 may obtain the first physiological data.

Optionally, the first physiological data may be second heart rate data collected by a second heart rate sensor. In addition to the second heart rate data, the first physiological data may further include other information.

Optionally, the second physiological data may alternatively be first heart rate data collected by a first heart rate sensor. In addition to the first heart rate data, the first physiological data may further include other information.

S803: The wearable device 100 determines a first blood pressure risk based on the pulse wave signal and the first physiological data at the first constant atmospheric pressure value.

After obtaining the pulse wave signal and the first physiological data at the first constant atmospheric pressure value, the wearable device 100 may determine the first blood pressure risk based on the pulse wave signal and the first physiological data at the first constant atmospheric pressure value.

The first blood pressure risk includes any one of the following: a high blood pressure risk, no risk, and a low blood pressure risk.

S804: The wearable device 100 determines a blood pressure value based on the first blood pressure risk and first pressure data, where the blood pressure value is related to the first blood pressure risk.

After the pulse wave signal and the first physiological data at the first constant atmospheric pressure value are obtained, the atmospheric pressure in the bulb may continue to linearly increase or continue to linearly decrease. After the atmospheric pressure in the bulb stops increasing or stops decreasing, the wearable device 100 may obtain the first pressure data in the inflation process and the deflation process of the bulb. The first pressure data may be used to determine a blood pressure value of a user.

Optionally, a plurality of models, for example, a model A, a model B, and a model C, are disposed in the wearable device 100. The model A, the model B, and the model C may obtain a blood pressure value based on pressure data. For example, the model A may be a high blood pressure model, the model A may be a normal blood pressure model, and the model C may be a low blood pressure model.

The wearable device 100 may determine, based on the first blood pressure risk, a model based on which the blood pressure value is determined.

When the first blood pressure risk is a high blood pressure risk, for example, blood pressure of the user is high, the wearable device 100 may input the first pressure data to the model A, and the model A outputs a blood pressure value, where the blood pressure value is high.

When the first blood pressure risk is no risk, for example, the blood pressure of the user falls within a normal range, the wearable device 100 may input the first pressure data to the model B, and the model B outputs a blood pressure value, where the blood pressure value falls within a normal blood pressure range.

When the first blood pressure risk is a low blood pressure risk, for example, the blood pressure of the user is low, the wearable device 100 may input the first pressure data to the model C, and the model C outputs a blood pressure value, where the blood pressure value is low.

Therefore, the wearable device 100 may determine the blood pressure value of the user based on the model corresponding to the blood pressure risk and the first pressure data, to improve accuracy of blood pressure measured based on the first blood pressure measurement method.

### (2) Measure blood pressure based on the second blood pressure measurement method

When preset time such as second time arrives, the wearable device 100 may prompt the user to start to measure blood pressure based on the second blood pressure measurement method.

For example, the wearable device 100 may display prompt information shown in FIG. 8I. The prompt information may include "Press the side button with one finger, and start to measure blood pressure". The prompt information is used to prompt the user to measure blood pressure based on the second blood pressure measurement method in a correct posture, to improve accuracy of blood pressure measured based on the second blood pressure measurement method.

### (3) Measure blood pressure based on the third blood pressure measurement method

When preset time such as third time arrives, the wearable device 100 may prompt the user to start to measure blood pressure based on the third blood pressure measurement method.

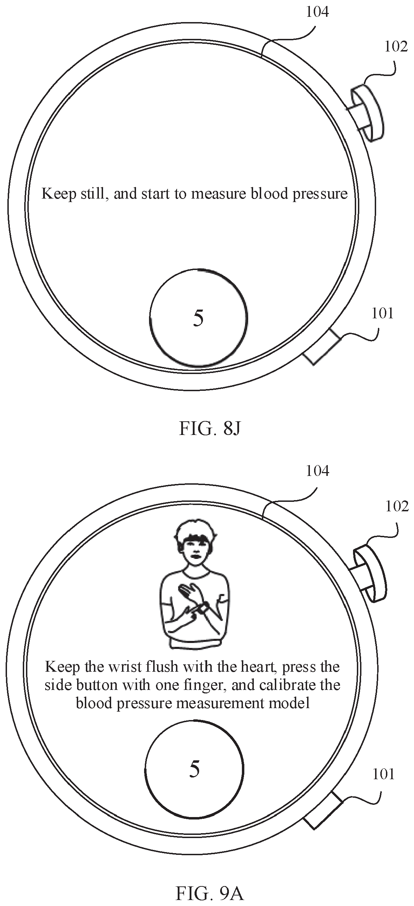

For example, the wearable device 100 may display prompt information shown in FIG. 8J. The prompt information may include "Keep still, and start to measure blood pressure". The prompt information is used to prompt the user to measure blood pressure based on the third blood pressure measurement method in a correct posture, to improve accuracy of blood pressure measured based on the third blood pressure measurement method.

### (4) Measure blood pressure based on the first blood pressure measurement method and the second blood pressure measurement method

When preset time such as fourth time arrives, the wearable device 100 may prompt the user to start to measure the blood pressure based on the first blood pressure measurement method and the second blood pressure measurement method.

In a possible implementation, the wearable device 100 may first display the prompt information shown in FIG. 8G, and prompt the user to measure the blood pressure based on the first blood pressure measurement method in a correct posture, for example, obtaining a blood pressure value H. After obtaining the blood pressure value H, the wearable device 100 then displays the prompt information shown in FIG. 8I, and prompt the user to measure the blood pressure based on the second blood pressure measurement method in a correct posture, for example, obtaining a blood pressure value I.

In another possible implementation, the wearable device 100 may first display the prompt information shown in FIG. 8I, and prompt the user to measure the blood pressure based on the second blood pressure measurement method in a correct posture. After obtaining the blood pressure value I, the wearable device 100 then displays the prompt information shown in FIG. 8G, and prompts the user to measure the blood pressure based on the first blood pressure measurement method in a correct posture, for example, obtaining the blood pressure value H.

The wearable device 100 may obtain a current blood pressure value of the user based on the blood pressure value H and the blood pressure value I. For example, the wearable device 100 may use an average value of the blood pressure value H and the blood pressure value I as the current blood pressure value of the user.

### (5) Measure blood pressure based on the first blood pressure measurement method and the third blood pressure measurement method

When preset time such as fifth time arrives, the wearable device 100 may prompt the user to start to measure the blood pressure based on the first blood pressure measurement method and the third blood pressure measurement method.

In a possible implementation, the wearable device 100 may first display the prompt information shown in FIG. 8G, and prompt the user to measure the blood pressure based on the first blood pressure measurement method in a correct posture, for example, obtaining the blood pressure value H. After obtaining the blood pressure value H, the wearable device 100 then displays the prompt information shown in FIG. 8J, and prompt the user to measure the blood pressure based on the third blood pressure measurement method in a correct posture, for example, obtaining a blood pressure value J.

In another possible implementation, the wearable device 100 may first display the prompt information shown in FIG. 8J, and prompt the user to measure the blood pressure based on the third blood pressure measurement method in a correct posture. After obtaining the blood pressure value J, the wearable device 100 then displays the prompt information shown in FIG. 8G, and prompts the user to measure the blood pressure based on the first blood pressure measurement method in a correct posture, for example, obtaining the blood pressure value H.

The wearable device 100 may obtain a current blood pressure value of the user based on the blood pressure value H and the blood pressure value J. For example, the wearable device 100 may use an average value of the blood pressure value H and the blood pressure value J as the current blood pressure value of the user.

### (6) Measure blood pressure based on the second blood pressure measurement method and the third blood pressure measurement method

When preset time such as sixth time arrives, the wearable device 100 may prompt the user to start to measure the blood pressure based on the second blood pressure measurement method and the third blood pressure measurement method.

In a possible implementation, the wearable device 100 may first display the prompt information shown in FIG. 8I, and prompt the user to measure the blood pressure based on the second blood pressure measurement method in a correct posture, for example, obtaining the blood pressure value I. After obtaining the blood pressure value I, the wearable device 100 then displays the prompt information shown in FIG. 8J, and prompt the user to measure the blood pressure based on the third blood pressure measurement method in a correct posture, for example, obtaining a blood pressure value J.

In another possible implementation, the wearable device 100 may first display the prompt information shown in FIG. 8J, and prompt the user to measure the blood pressure based on the third blood pressure measurement method in a correct posture. After obtaining the blood pressure value J, the wearable device 100 then displays the prompt information shown in FIG. 8I, and prompt the user to measure the blood pressure based on the second blood pressure measurement method in a correct posture, for example, obtaining the blood pressure value I.

The wearable device 100 may obtain a current blood pressure value of the user based on the blood pressure value I and the blood pressure value J. For example, the wearable device 100 may use an average value of the blood pressure value I and the blood pressure value J as the current blood pressure value of the user.

### (7) Measure blood pressure based on the first blood pressure measurement method, the second blood pressure measurement method, and the third blood pressure measurement method

When preset time such as seventh time arrives, the wearable device 100 may prompt the user to start to measure the blood pressure based on the first blood pressure measurement method, the second blood pressure measurement method, and the third blood pressure measurement method.

The wearable device 100 may display the prompt information shown in FIG. 8G, and obtain the blood pressure value H; display the prompt information shown in FIG. 8I, and obtain the blood pressure value I; and display the prompt information shown in FIG. 8J, and obtain the blood pressure value J. The foregoing three types of prompt information may be displayed in any sequence. This is not limited in this application.

The wearable device 100 may obtain a current blood pressure value of the user based on the blood pressure value H, the blood pressure value I, and the blood pressure value J. For example, the wearable device 100 may use an average value of the blood pressure value H, the blood pressure value I, and the blood pressure value J as the current blood pressure value of the user.

### 2. Unawareness scenario

**The unawareness scenario may include an unawareness calibration scenario and an unawareness measurement scenario.**

### (1) Calibrate the second target model of the second blood pressure measurement method

### A. Initial calibration of the second target model

A principle of initial calibration of the second target model in the unawareness scenario is similar to the principle of initial calibration of the second target model in the awareness scenario. For details, refer to specific descriptions of initial calibration of the second target model in the awareness scenario. Details are not described herein again in this application.

A difference lies in that, in the unawareness scenario, when initially calibrating the second target model, the wearable device 100 only needs to prompt the user to maintain one posture to complete a model calibration process. In this way, the user can complete the model calibration process without changing the posture, which reduces user operations.

For example, when the second target model starts to be calibrated, the wearable device 100 may prompt the user to maintain a posture shown in FIG. 9A, that is, keeping the wrist wearing the wearable device 100 flush with the heart, and pressing the side button with one finger.

When the user maintains the posture shown in FIG. 9A, the wearable device 100 collects third information based on the first blood pressure measurement method, collects second information based on the second blood pressure measurement method, and collects fourth information based on the first blood pressure measurement method. The third information, the second information, and the fourth information may be obtained in any sequence. This is not limited.

In this way, the user may obtain a plurality of pieces of information in one posture, and the user is unaware of a sequence of obtaining the plurality of pieces of information. This implements unawareness calibration of the second target model.

After the second information, the third information, and the fourth information are obtained, for how the wearable device 100 calibrates the second target model, refer to detailed descriptions of initial calibration of the second target model in the unawareness scenario. Details are not described herein again in this application.

In some embodiments, when determining, based on the motion data collected by the motion sensor, whether the user is in a static state or a non-static state, the wearable device 100 may also collect the third information or the fourth information based on the first blood pressure measurement method for one time. A sequence of obtaining the third information or the fourth information and the second information is not limited.

### B. Non-initial calibration of the second target model

A principle of non-initial calibration of the second target model in the unawareness scenario is similar to a principle of initial calibration of the second target model in the unawareness scenario. A difference lies in that, in the unawareness scenario, when performing non-initial calibration on the second target model, the wearable device 100 needs to collect data for one time based on the first blood pressure measurement method. For example, the wearable device 100 needs to collect third information based on the first blood pressure measurement method, to calibrate the second target model. For details, refer to specific descriptions of initial calibration of the second target model in the unawareness scenario. Details are not described herein again in this application.

### (2) Calibrate the first target model of the third blood pressure measurement method

### A. Initial calibration of the first target model

In some embodiments, the wearable device 100 may calibrate the first target model based on a blood pressure value and/or pressure data collected based on the first blood pressure measurement method.

A principle of initial calibration of the first target model based on the blood pressure value and/or the pressure data collected based on the first blood pressure measurement method in the unawareness scenario is similar to the principle of initial calibration of the first target model based on the blood pressure value and/or the pressure data collected based on the first blood pressure measurement method in the awareness scenario. For details, refer to specific descriptions of initial calibration of the first target model in the awareness scenario. Details are not described herein again in this application.

A difference lies in that, in the unawareness scenario, when initially calibrating the first target model, the wearable device 100 only needs to prompt the user to maintain one posture to complete a model calibration process. In this way, the user can complete the model calibration process without changing the posture, which reduces user operations.

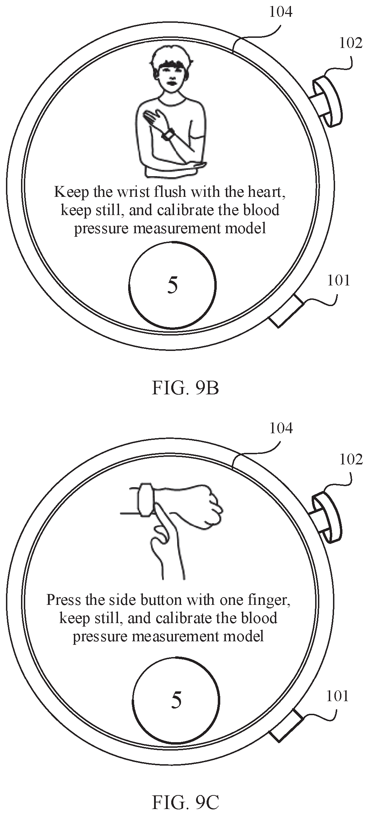

For example, when the first target model starts to be calibrated, the wearable device 100 may prompt the user to maintain a posture shown in FIG. 9B, that is, keeping the wrist wearing the wearable device 100 flush with the heart, and keeping still.

When the user maintains the posture shown in FIG. 9B, the wearable device 100 collects third information based on the first blood pressure measurement method, collects fifth information based on the third blood pressure measurement method, and collects fourth information based on the first blood pressure measurement method. The third information, the fifth information, and the fourth information may be obtained in any sequence. This is not limited.

In this way, the user may obtain a plurality of pieces of information in one posture, and the user is unaware of a sequence of obtaining the plurality of pieces of information. This implements unawareness calibration of the second target model.

After the fifth information, the third information, and the fourth information are obtained, for how the wearable device 100 calibrates the first target model, refer to detailed descriptions of initial calibration of the first target model in the unawareness scenario. Details are not described herein again in this application.

In some embodiments, when determining, based on the motion data collected by the motion sensor, whether the user is in a static state or a non-static state, the wearable device 100 may also collect the third information or the fourth information based on the first blood pressure measurement method for one time. A sequence of obtaining the third information or the fourth information and the fifth information is not limited.

In another embodiment, the wearable device 100 may calibrate the first target model based on a blood pressure value and/or pressure data collected based on the second blood pressure measurement method.

A principle of initial calibration of the first target model based on the blood pressure value collected based on the second blood pressure measurement method in the unawareness scenario is similar to the principle of initial calibration of the first target model based on the blood pressure value collected based on the second blood pressure measurement method in the awareness scenario. For details, refer to specific descriptions of initial calibration of the first target model in the awareness scenario. Details are not described herein again in this application.

A difference lies in that, in the unawareness scenario, when initially calibrating the first target model, the wearable device 100 only needs to prompt the user to maintain one posture to complete a model calibration process. In this way, the user can complete the model calibration process without changing the posture, which reduces user operations.

For example, when the first target model starts to be calibrated, the wearable device 100 may prompt the user to maintain a posture shown in FIG. 9C, that is, pressing the side button with one finger, and keeping still.

When the user maintains the posture shown in FIG. 9C, the wearable device 100 collects sixth information based on the second blood pressure measurement method, collects fifth information based on the third blood pressure measurement method, and collects seventh information based on the second blood pressure measurement method. The sixth information, the fifth information, and the seventh information may be obtained in any sequence. This is not limited.

In this way, the user may obtain a plurality of pieces of information in one posture, and the user is unaware of a sequence of obtaining the plurality of pieces of information. This implements unawareness calibration of the second target model.

After the fifth information, the sixth information, and the seventh information are obtained, for how the wearable device 100 calibrates the first target model, refer to detailed descriptions of initial calibration of the first target model in the unawareness scenario. Details are not described herein again in this application.

In some embodiments, when determining, based on the motion data collected by the motion sensor, whether the user is in the static state or the non-static state, the wearable device 100 may also collect the sixth information or the seventh information based on the second blood pressure measurement method for one time. A sequence of obtaining the sixth information or the seventh information and the fifth information is not limited.

In another implementation, when initially calibrating the second target model, the wearable device 100 may also automatically calibrate the first target model based on the third information and the fourth information that are collected based on the first blood pressure measurement method.

### B. Non-initial calibration of the first target model

A principle of non-initial calibration of the first target model in the unawareness scenario is similar to a principle of initial calibration of the first target model in the unawareness scenario. A difference lies in that, in the unawareness scenario, when performing non-initial calibration on the first target model, the wearable device 100 only needs to collect data for one time based on the first blood pressure measurement method or collect data for one time based on the second blood pressure measurement method. For example, the wearable device 100 only needs to collect third information based on the first blood pressure measurement method or collect sixth information based on the second blood pressure measurement method, to calibrate the first target model. For details, refer to specific descriptions of initial calibration of the first target model in the unawareness scenario. Details are not described herein again in this application.

In another implementation, when measuring blood pressure of the user based on the first blood pressure measurement method, the wearable device 100 may also automatically calibrate the first target model based on the third information collected based on the first blood pressure measurement method.

In another implementation, when measuring blood pressure of the user based on the second blood pressure measurement method, the wearable device 100 may also automatically calibrate the first target model based on the sixth information obtained based on the second blood pressure measurement method.

### 2. Unawareness blood pressure measurement scenario

### (1) Measure blood pressure based on the first blood pressure measurement method

When preset time such as first time arrives, the wearable device 100 may measure the blood pressure based on the first blood pressure measurement method, for example, display the prompt information shown in FIG. 8G, and prompt the user to measure the blood pressure based on the third blood pressure measurement method.

Optionally, alternatively, the wearable device 100 may automatically start to measure blood pressure based on the first blood pressure measurement method without prompting the user.

### (2) Measure blood pressure based on the second blood pressure measurement method

When preset time such as second time arrives, the wearable device 100 may measure the blood pressure based on the second blood pressure measurement method, for example, display the prompt information shown in FIG. 8I, and prompt the user to measure the blood pressure based on the third blood pressure measurement method.

Optionally, alternatively, the wearable device 100 may automatically start to measure blood pressure based on the second blood pressure measurement method without prompting the user.

### (3) Measure blood pressure based on the third blood pressure measurement method

When preset time such as third time arrives, the wearable device 100 may measure the blood pressure based on the third blood pressure measurement method, for example, display the prompt information shown in FIG. 8J, and prompt the user to measure the blood pressure based on the third blood pressure measurement method.

Optionally, alternatively, the wearable device 100 may automatically start to measure blood pressure based on the third blood pressure measurement method without prompting the user.

### (4) Measure blood pressure based on the first blood pressure measurement method and the second blood pressure measurement method

When preset time such as fourth time arrives, the wearable device 100 may measure the blood pressure based on the first blood pressure measurement method and the second blood pressure measurement method.

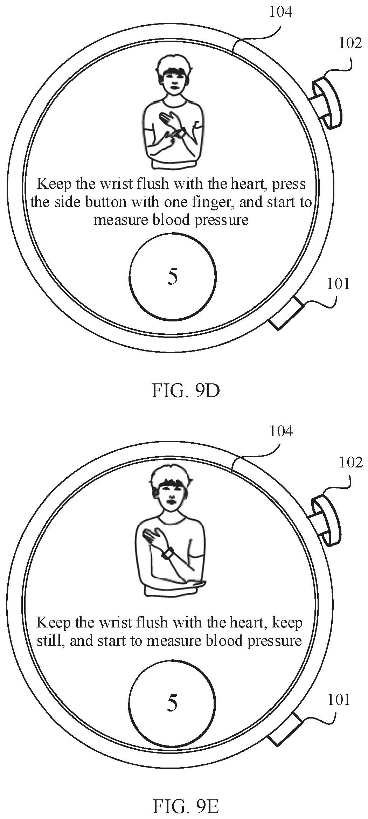

For example, the wearable device 100 may display prompt information shown in FIG. 9D, that is, keeping the wrist wearing the wearable device 100 flush with the heart, pressing the side button with one finger, and starting to measure blood pressure.

When the user maintains the posture shown in FIG. 9D, the wearable device 100 may obtain a blood pressure value H based on the first blood pressure measurement method, and obtain a blood pressure value I based on the second blood pressure measurement method. A sequence of obtaining the blood pressure value H and obtaining the blood pressure value I is not limited. In this way, the user may obtain the blood pressure value of the user in one posture based on two blood pressure measurement methods.

The wearable device 100 may obtain a current blood pressure value of the user based on the blood pressure value H and the blood pressure value I. For example, the wearable device 100 may use an average value of the blood pressure value H and the blood pressure value I as the current blood pressure value of the user.

### (5) Measure blood pressure based on the first blood pressure measurement method and the third blood pressure measurement method

When preset time such as fourth time arrives, the wearable device 100 may measure the blood pressure based on the first blood pressure measurement method and the second blood pressure measurement method.

For example, the wearable device 100 may display prompt information shown in FIG. 9E, that is, keeping the wrist wearing the wearable device 100 flush with the heart, keeping still, and starting to measure blood pressure.

When the user maintains the posture shown in FIG. 9E, the wearable device 100 may obtain the blood pressure value H based on the first blood pressure measurement method, and obtain a blood pressure value J based on the third blood pressure measurement method. A sequence of obtaining the blood pressure value H and obtaining the blood pressure value J is not limited. In this way, the user may obtain the blood pressure value of the user in one posture based on two blood pressure measurement methods.

The wearable device 100 may obtain a current blood pressure value of the user based on the blood pressure value H and the blood pressure value J. For example, the wearable device 100 may use an average value of the blood pressure value H and the blood pressure value J as the current blood pressure value of the user.

### (6) Measure blood pressure based on the second blood pressure measurement method and the third blood pressure measurement method

When preset time such as sixth time arrives, the wearable device 100 may measure the blood pressure based on the second blood pressure measurement method and the third blood pressure measurement method.

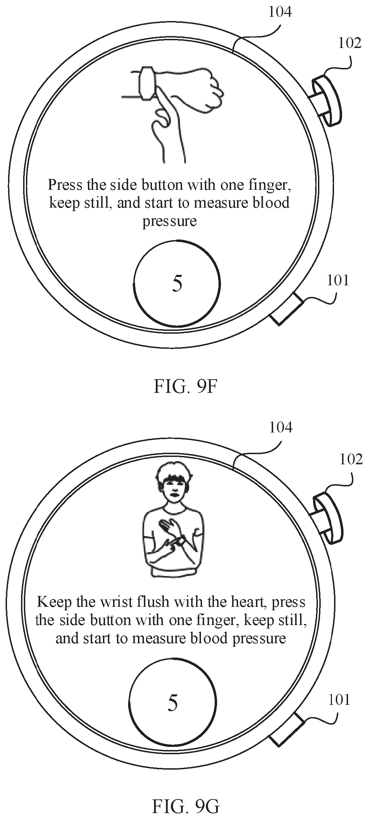

For example, the wearable device 100 may display prompt information shown in FIG. 9F, that is, pressing the side button with one finger, keeping still, and starting to measure blood pressure.

When the user maintains the posture shown in FIG. 9F, the wearable device 100 may obtain the blood pressure value I based on the second blood pressure measurement method, and obtain the blood pressure value J based on the third blood pressure measurement method. A sequence of obtaining the blood pressure value I and obtaining the blood pressure value J is not limited. In this way, the user may obtain the blood pressure value of the user in one posture based on two blood pressure measurement methods.

The wearable device 100 may obtain a current blood pressure value of the user based on the blood pressure value I and the blood pressure value J. For example, the wearable device 100 may use an average value of the blood pressure value I and the blood pressure value J as the current blood pressure value of the user.

### (7) Measure blood pressure based on the first blood pressure measurement method, the second blood pressure measurement method, and the third blood pressure measurement method

When preset time such as seventh time arrives, the wearable device 100 may measure the blood pressure based on the first blood pressure measurement method, the second blood pressure measurement method, and the third blood pressure measurement method.

For example, the wearable device 100 may display prompt information shown in FIG. 9G, that is, keeping the wrist wearing the wearable device 100 flush with the heart, pressing the side button with one finger, keeping still, and starting to measure blood pressure.

When the user maintains the posture shown in FIG. 9G, the wearable device 100 may obtain the blood pressure value H based on the first blood pressure measurement method, obtain the blood pressure value I based on the second blood pressure measurement method, and obtain the blood pressure value J based on the third blood pressure measurement method. A sequence of obtaining the blood pressure value H, obtaining the blood pressure value I and obtaining the blood pressure value J is not limited. In this way, the user may obtain the blood pressure value of the user in one posture based on three blood pressure measurement methods.

The wearable device 100 may obtain a current blood pressure value of the user based on the blood pressure value H, the blood pressure value I, and the blood pressure value J. For example, the wearable device 100 may use an average value of the blood pressure value H, the blood pressure value I, and the blood pressure value J as the current blood pressure value of the user.

It should be noted that, when the first target model or the second target is calibrated, the foregoing embodiment is described by using an example in which the user is prompted to perform calibration. In another embodiment, alternatively, the wearable device 100 may internally calibrate the first target model or the second target model, and does not prompt the user to perform calibration.

The above descriptions are only some embodiments and implementations of this application, and the protection scope of this application is not limited to this. Any variation or replacement readily figured out by a person skilled in the art within the technical scope disclosed in this application shall fall within the protection scope of this application. Therefore, the protection scope of this application shall be subject to the protection scope of the claims.

It may be understood that each user interface described in embodiments of this application is merely an example interface, and constitutes no limitation on the solutions of this application. In another embodiment, the user interface may use different interface layouts, may include more or fewer controls, and may add or reduce other function options, and provided that the user interface is based on a same inventive idea provided in this application, all fall within the protection scope of this application.

It should be noted that, if no contradiction or conflict occurs, any feature or any part of any feature in any embodiment of this application may be combined, and a combined technical solution also falls within the scope of embodiments of this application.

In conclusion, the foregoing embodiments are merely intended for describing the technical solutions of this application, but not for limiting this application. Although this application is described in detail with reference to the foregoing embodiments, persons of ordinary skill in the art should understand that they may still make modifications to the technical solutions described in the foregoing embodiments or make equivalent replacements to some technical features thereof, without departing from the scope of the technical solutions of the embodiments of this application.

## Claims

1. A blood pressure measurement method, wherein a wearable device comprises a first heart rate sensor and a second heart rate sensor, the wearable device further comprises a watch body, the watch body comprises a case back and a display surface, a first button is disposed on a side surface of the watch body, the first heart rate sensor is disposed on the first button, and the second heart rate sensor is disposed on the case back; and the method comprises:
obtaining, by the wearable device, first information, wherein the first information comprises one or more of the following: user information, environment information, and motion data; and
determining, by the wearable device, a first blood pressure measurement solution based on the first information, wherein the first blood pressure measurement solution comprises at least one of the following blood pressure measurement methods: a second blood pressure measurement method for measuring blood pressure based on first heart rate data collected by the first heart rate sensor, and a third blood pressure measurement method for measuring blood pressure based on second heart rate data collected by the second heart rate sensor.

2. The method according to claim 1, wherein the first blood pressure measurement solution further comprises any one or more of the following: a quantity of times of the second blood pressure measurement method, a quantity of times of the third blood pressure measurement method, measurement time of the second blood pressure measurement method, and measurement time of the third blood pressure measurement method.

3. The method according to claim 1 or 2, wherein the user information comprises one or more of the following: blood pressure, a heart rate, a body temperature, a pulse, a respiratory rate, blood glucose, a smoking history, a drinking history, a history of disease, a medication history, lifestyle habits, gender, age, a height, and a weight; and
the environment information comprises any one or more of the following: climate, a temperature, altitude, season, and weather.

4. The method according to any one of claims 1 to 3, wherein after determining, by the wearable device, the first blood pressure measurement solution based on the first information, the method further comprises:
prompting, by the wearable device, a user to view content of the first blood pressure measurement solution.

5. The method according to any one of claims 1 to 4, wherein after determining, by the wearable device, the first blood pressure measurement solution based on the first information, the method further comprises:
obtaining, by the wearable device, a first blood pressure value through measurement; and
obtaining, by the wearable device, a second blood pressure measurement solution based on the first blood pressure value.

6. The method according to claim 5, wherein if the first blood pressure value is high or low, a quantity of execution times of the second blood pressure measurement method in the second blood pressure measurement solution is greater than that of the second blood pressure measurement method in the first blood pressure measurement solution.

7. The method according to claim 5 or 6, wherein if the first blood pressure value is normal, the quantity of execution times of the second blood pressure measurement method in the second blood pressure measurement solution is less than or equal to that of the second blood pressure measurement method in the first blood pressure measurement solution.

8. The method according to any one of claims 1 to 7, wherein after determining, by the wearable device, the blood pressure measurement solution based on the first information, the method further comprises:
measuring, by the wearable device, blood pressure based on the first blood pressure measurement solution, to obtain a plurality of blood pressure values; and
determining, by the wearable device, a third blood pressure measurement solution based on the plurality of blood pressure values, wherein the third blood pressure measurement solution is different from the first blood pressure measurement solution.

9. The method according to any one of claims 1 to 8, wherein after determining, by the wearable device, the blood pressure measurement solution based on the first information, the method further comprises:
obtaining, by the wearable device, a third blood pressure value based on the second blood pressure measurement method;
collecting, by the wearable device, the second heart rate data based on the second heart rate sensor; and
updating, by the wearable device, a first target model based on the third blood pressure value and the second heart rate data, wherein a fifth blood pressure value obtained by the updated first target model based on the second heart rate data is closer to the third blood pressure value than a sixth blood pressure value obtained based on the second heart rate data by the first target model before the update.

10. The method according to any one of claims 1 to 9, wherein the wearable device is a watch or a band.

11. The method according to any one of claims 1 to 10, wherein the wearable device further comprises an electrocardiogram sensor and a pressure sensor, the electrocardiogram sensor comprises a plurality of electrodes, the plurality of electrodes are disposed on the first button and the case back, and the pressure sensor is disposed on the first button.

12. The method according to claim 11, wherein measuring the blood pressure based on the second blood pressure measurement method comprises:
obtaining, by the pressure sensor, a first pressure value on the first button.

13. The method according to claim 12, wherein measuring the blood pressure based on the second blood pressure measurement method further comprises:
prompting, based on the first pressure value, the user to increase or decrease pressing force on the first button.

14. The method according to claim 13, wherein the prompting, based on the first pressure value, the user to increase or decrease the pressing force on the first button specifically comprises:
when it is detected that the first pressure value is greater than a first threshold, prompting the user to decrease the pressing force on the heart rate sensor; and
when it is detected that the first pressure value is less than a second threshold, prompting the user to increase the pressing force on the heart rate sensor.

15. The method according to claim 14, wherein measuring the blood pressure based on the second blood pressure measurement method further comprises:
displaying a pressure indicator bar, wherein the pressure indicator bar indicates a pressure value, on the first button, obtained by the pressure sensor.

16. The method according to any one of claims 11 to 15, wherein measuring the blood pressure based on the second blood pressure measurement method comprises:
obtaining a second blood pressure value based on the first pressure value, the first heart rate data, and electrocardiogram data collected by the electrocardiogram sensor.

17. The method according to claim 16, wherein when the pressure value on the first button meets a preset pressure range, the first heart rate data is heart rate data collected by the first heart rate sensor, and the electrocardiogram data is electrocardiogram data collected by the electrocardiogram sensor.

18. The method according to claim 17, wherein the preset pressure range is from 40 gF to 70 gF.

19. A wearable device, wherein the wearable device comprises a memory and a processor, the memory is coupled to the processor, the memory is configured to store a computer program, and when the processor invokes the computer program, the wearable device is enabled to perform the method according to any one of claims 1 to 18.

20. A computer-readable storage medium, comprising instructions, wherein when the instructions are run on a wearable device, the wearable device is enabled to perform the method according to any one of claims 1 to 18.
